# EUROPEAN PATENT APPLICATION

(11) **EP 4 656 193 A1**
(43) Date of publication of application: **03.12.2025**
(21) Application number: 23932127.6
(22) Date of filing: 03.10.2023
(51) Int. Cl.: A61K 31/5025, A61K 31/506, A61K 31/519, A61K 45/00, A61K 45/06, A61P 35/00, A61P 43/00

(54) **PHARMACEUTICAL COMPOSITION FOR TREATING TUMORS**

(30) Priority: 06.04.2023 JP 2023061941
(71) Applicant: Eisai R&D Management Co., Ltd., Tokyo 112-8088 (JP)
(72) Inventor: OZAWA Yoichi, Tsukuba-shi, Ibaraki 300-2635 (JP); KATO Yu, Tsukuba-shi, Ibaraki 300-2635 (JP); ADACHI Yusuke, Tsukuba-shi, Ibaraki 300-2635 (JP)
(74) Representative: Hoffmann Eitle
(86) International application number: PCT/JP2023/036011
(87) International publication number: WO 2024/209717

(57) **Abstract**

Disclosed is a pharmaceutical composition for treating tumors, which comprises (6S,9aS)-N-benzyl-8-({6-[3-(4-ethylpiperazin-1-yl)azetidin-1-yl]pyridin-2-yl}methyl)-6-(2-fluoro-4-hydroxybenzyl)-4,7-dioxo-2-(prop-2-en-l-yl)hexahydro-2H-pyrazino[2,1-c][1,2,4]triazine-1(6H)-carboxamide represented by formula (I) or a pharmaceutically acceptable salt thereof and is administered in combination with an MEK inhibitor.

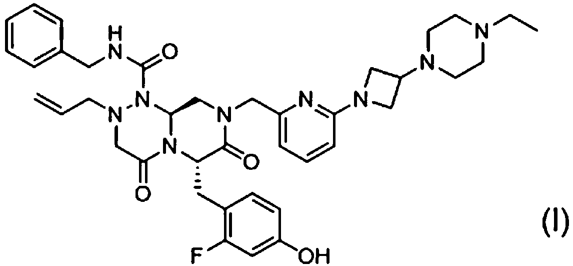

## Description

### Technical Field

The present invention relates to a pharmaceutical composition for treating tumors.

### Background Art

A Mitogen activated protein kinase (MAPK) pathway is a signaling pathway highly conserved in yeast to mammals, and regulates various cellular functions such as cell proliferation, survival, and differentiation. It is known that a RAS-RAF-MEK-ERK signaling pathway, which is one of the main MAPK pathways, is constitutively activated in many tumors and contributes to cancer cell proliferation and the like, and drug discovery research has been conducted using a constituent molecule thereof as a target candidate for cancer treatment (Non Patent Literature 1).

As one of agents targeting the RAS-RAF-MEK-ERK signaling pathway, a MEK inhibitor is known. For example, Trametinib, which was approved as a first MEK inhibitor in the world in 2013, is an orally administrable molecular targeting drug, and is used singly or in combination with Dabrafenib (BRAF inhibitor) for treatment of BRAF gene mutation-positive unresectable or metastatic malignant melanoma.

On the other hand, a Wnt/β-catenin signaling pathway is a signaling pathway highly conserved in an evolution process of animals, and regulates gene expression related to proliferation and differentiation of cells, formation of a body axis and an organ, and the like. It has been reported that the Wnt/β-catenin signaling pathway and the RAS-RAF-MEK-ERK signaling pathway form a positive feedback loop by mutual crosstalk, thereby contributing to cancer cell proliferation, and it is important for an antitumor effect to inhibit both pathways simultaneously (Non Patent Literature 2).

Regarding the antitumor effect by inhibition of the Wnt/β-catenin signaling pathway and the RAS-RAF-MEK-ERK signaling pathway, Non Patent Literature 3 describes that in a mouse model subcutaneously transplanted with human colorectal cancer cell line HT-29 in which a MAPK pathway is activated by BRAF V600E-mutant, when ICG001 which is a Wnt/β-catenin signaling pathway inhibitor is administered either (i) in combination with Vemurafenib, a BRAF inhibitor, or (ii) in combination with both Vemurafenib and Trametinib, the antitumor effect is enhanced compared to the single administration. Furthermore, Non Patent Literature 4 describes that in a PDX model mouse produced by subcutaneously transplantation with a patient-derived colorectal cancer organoid, when ICG001 is administered in combination with Trametinib, the antitumor effect is enhanced compared to the single administration.

Here, as a compound having a Wnt Pathway modulating action, (6S,9aS)-N-benzyl-8-((6-(3-(4-ethylpiperazin-1-yl)azetidine-1-yl)pyridin-2-yl)methyl)-6-((2-fluoro-4-hydroxyphenyl)methyl)-4,7-dioxo-2-(prop-2-en-1-yl)-octahydro-1H-pyrazino[2,1-c][1,2,4]triazine-1-carboxamide represented by the following formula (X) (Patent Literature 1), (6S,9aS)-N-benzyl-8-({6-[3-(4-ethylpiperazin-1-yl)azetidin-1-yl]pyridin-2-yl}methyl)-6-(2-fluoro-4-hydroxybenzyl)-4,7-dioxo-2-(prop-2-en-1-yl)hexahydro-2H-pyrazino[2,1-c][1,2,4]triazine-1(6H)-carboxamide represented by the following formula (X) (Patent Literature 2), and E7386 represented by the following formula (I) are known (Non Patent Literature 5).

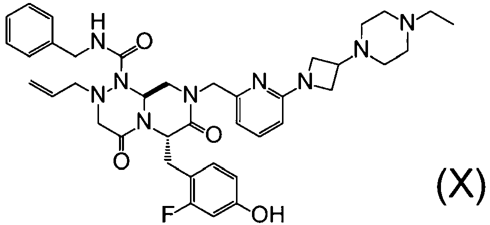

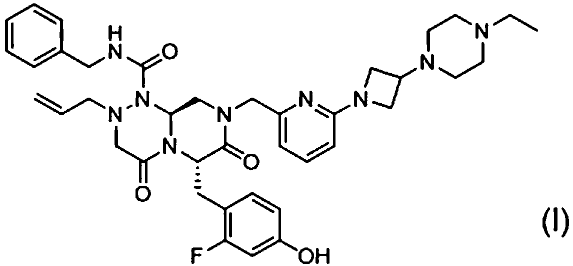

(6S,9aS)-N-benzyl-8-((6-(3-(4-ethylpiperazin-1-yl)azetidine-1-yl)pyridin-2-yl)methyl)-6-((2-fluoro-4-hydroxyphenyl)methyl)-4,7-dioxo-2-(prop-2-en-1-yl)-octahydro-1H-pyrazino[2,1-c][1,2,4]triazine-1-carboxamide disclosed in Patent Literature 1 and (6S,9aS)-N-benzyl-8-({6-[3-(4-ethylpiperazin-1-yl)azetidin-1-yl]pyridin-2-yl}methyl)-6-(2-fluoro-4-hydroxybenzyl)-4,7-dioxo-2-(prop-2-en-1-yl)hexahydro-2H-pyrazino[2,1-c][1,2,4]triazine-1(6H)-carboxamide disclosed in Patent Literature 2 both represent a compound represented by formula (I) in IUPAC nomenclature.

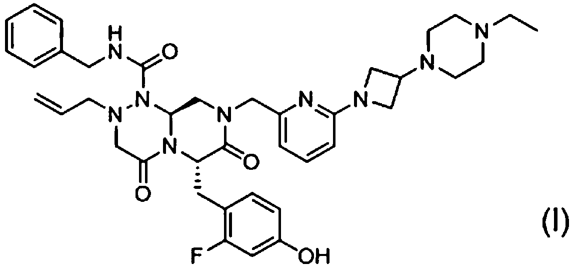

The notation method of the chemical bond in structural formulas of formula (X) and formula (I) are different, but they represent the same stereochemistry. Therefore, in the present specification, formula (I) is used as a structural formula, and (6S,9aS)-N-benzyl-8-({6-[3-(4-ethylpiperazin-1-yl)azetidin-1-yl]pyridin-2-yl}methyl)-6-(2-fluoro-4-hydroxybenzyl)-4,7-dioxo-2-(prop-2-en-1-yl)hexahydro-2H-pyrazino[2,1-c][1,2,4]triazine-1(6H)-carboxamide is used as a chemical name.

### Citation List

### Patent Literature

Patent Literature 1: WO 2015/098853 A
Patent Literature 2: WO 2016/208576 A

### Non Patent Literature

Non Patent Literature 1: Nobuya Ishii, "Progress of research and development of MAPK pathway inhibitors.", Folia Pharmacol. Jpn. 141, 15 to 21 (2013).
Non Patent Literature 2: D. Kim et. al., "A hidden oncogenic positive feedback loop caused by crosstalk between Wnt and ERK pathway.", Oncogene 26, 4571-9 (2007).
Non Patent Literature 3: Chen G. et al., "Wnt/β-Catenin Pathway Activation Mediates Adaptive Resistance to BRAF Inhibition in Colorectal Cancer.", Mol. Cancer Ther. 17(4), 806-813 (2018).
Non Patent Literature 4: Zhan T. et al., "MEK inhibitors activate Wnt signalling and induce stem cell plasticity in colorectal cancer" Nat. Commun. 10, 2197 (2019).
Non Patent Literature 5: Kazuhiro Yamada et al., "E7386, a Selective Inhibitor of the Interaction between β-Catenin and CBP, Exerts Antitumor Activity in Tumor Models with Activated Canonical Wnt Signaling.", Cancer Res. 81 (4), 1052-1062 (2021).

### Summary of Invention

### Technical Problem

Although the combined use of ICG001 and a MEK inhibitor, which has been reported so far, suppresses tumor growth, the suppression effect of the combination is not sufficient. In addition, there is no known combination therapy using the compound represented by formula (I) and the MEK inhibitor.

An object of the present invention is to provide a new pharmaceutical composition for treating tumors.

### Solution to Problem

As a result of intensive studies, the present inventors have found that combined administration of (6S,9aS)-N-benzyl-8-({6-[3-(4-ethylpiperazin-1-yl)azetidin-1-yl]pyridin-2-yl}methyl)-6-(2-fluoro-4-hydroxybenzyl)-4,7-dioxo-2-(prop-2-en-1-yl)hexahydro-2H-pyrazino[2,1-c][1,2,4]triazine-1(6H)-carboxamide (hereinafter, also referred to as a compound represented by formula (I)) or a pharmaceutically acceptable salt thereof and a MEK inhibitor exhibits a high antitumor effect, and have completed the present invention.

That is, the present disclosure relates to the following.
[1] A pharmaceutical composition for treating a tumor or a therapeutic agent for a tumor, comprising (6S,9aS)-N-benzyl-8-({6-[3-(4-ethylpiperazin-1-yl)azetidin-1-yl]pyridin-2-yl} methyl)-6-(2-fluoro-4-hydroxybenzyl)-4,7-dioxo-2-(prop-2-en-1-yl)hexahydro-2H-pyrazino[2,1-c][1,2,4]triazine-1(6H)-carboxamide represented by the following formula (I) 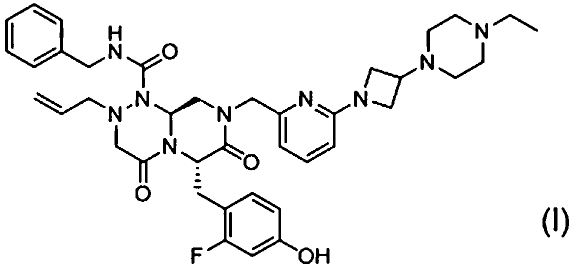 or a pharmaceutically acceptable salt thereof,
   wherein the composition or the agent is administered in combination with a MEK inhibitor.
[2] A pharmaceutical composition for treating a tumor or a therapeutic agent for a tumor, comprising a MEK inhibitor,
   wherein the composition or the agent is administered in combination with (6S,9aS)-N-benzyl-8-({6-[3-(4-ethylpiperazin-1-yl)azetidin-1-yl]pyridin-2-yl}methyl)-6-(2-fluoro-4-hydroxybenzyl)-4,7-dioxo-2-(prop-2-en-1-yl)hexahydro-2H-pyrazino[2,1-c][1,2,4]triazine-1(6H)-carboxamide represented by the following formula (I) 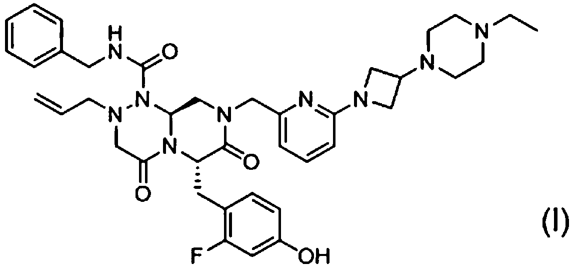 or a pharmaceutically acceptable salt thereof.
[3] A method of treating a tumor, wherein
   an effective amount of (6S,9aS)-N-benzyl-8-({6-[3-(4-ethylpiperazin-1-yl)azetidin-1-yl]pyridin-2-yl}methyl)-6-(2-fluoro-4-hydroxybenzyl)-4,7-dioxo-2-(prop-2-en-1-yl)hexahydro-2H-pyrazino[2,1-c][1,2,4]triazine-1(6H)-carboxamide represented by the following formula (I) 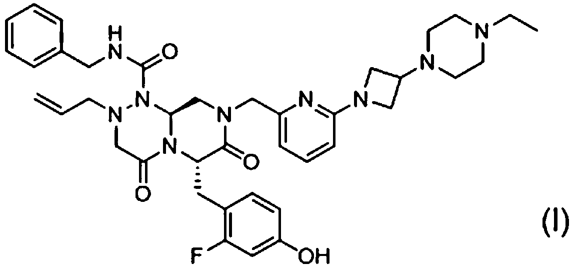
   or a pharmaceutically acceptable salt thereof and an effective amount of a MEK inhibitor are administered to a patient in combination.
[4] Use of (6S,9aS)-N-benzyl-8-({6-[3-(4-ethylpiperazin-1-yl)azetidin-1-yl]pyridin-2-yl}methyl)-6-(2-fluoro-4-hydroxybenzyl)-4,7-dioxo-2-(prop-2-en-1-yl)hexahydro-2H-pyrazino[2,1-c][1,2,4]triazine-1(6H)-carboxamide represented by the following formula (I) 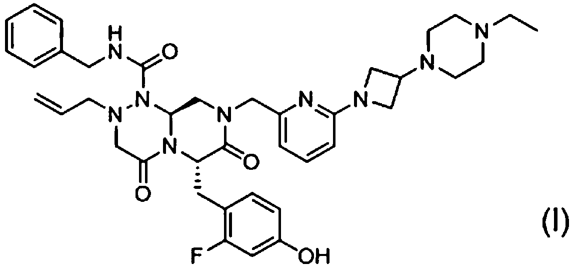
   or a pharmaceutically acceptable salt thereof
   in the manufacture of a pharmaceutical composition for treating a tumor or a therapeutic agent for a tumor,
   wherein the composition or the agent is administered in combination with a MEK inhibitor.
[5] Use of a MEK inhibitor in the manufacture of a pharmaceutical composition for treating a tumor or a therapeutic agent for a tumor, wherein the composition or the agent is administered in combination with (6S,9aS)-N-benzyl-8-({6-[3-(4-ethylpiperazin-1-yl)azetidin-1-yl]pyridin-2-yl} methyl)-6-(2-fluoro-4-hydroxybenzyl)-4,7-dioxo-2-(prop-2-en-1-yl)hexahydro-2H-pyrazino[2,1-c][1,2,4]triazine-1(6H)-carboxamide represented by the following formula (I) 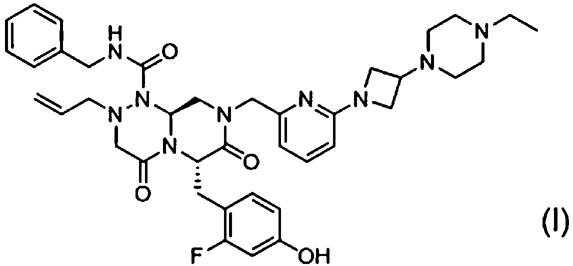 or a pharmaceutically acceptable salt thereof.
[6] (6S,9aS)-N-benzyl-8-({6-[3-(4-ethylpiperazin-1-yl)azetidin-1-yl]pyridin-2-yl} methyl)-6-(2-fluoro-4-hydroxybenzyl)-4,7-dioxo-2-(prop-2-en-1-yl)hexahydro-2H-pyrazino[2,1-c][1,2,4]triazine-1(6H)-carboxamide represented by the following formula (I) 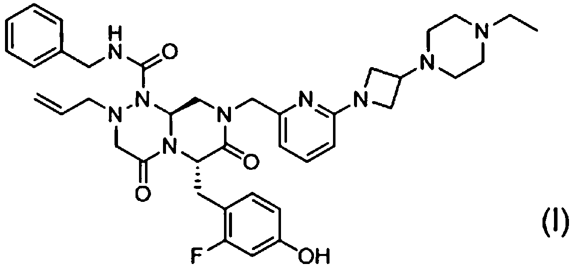 or a pharmaceutically acceptable salt thereof for use in the treatment of a tumor in combination with a MEK inhibitor.
[7] A MEK inhibitor for use in the treatment of a tumor in combination with (6S,9aS)-N-benzyl-8-({6-[3-(4-ethylpiperazin-1-yl)azetidin-1-yl]pyridin-2-yl} methyl)-6-(2-fluoro-4-hydroxybenzyl)-4,7-dioxo-2-(prop-2-en-1-yl)hexahydro-2H-pyrazino[2,1-c][1,2,4]triazine-1(6H)-carboxamide represented by the following formula (I) 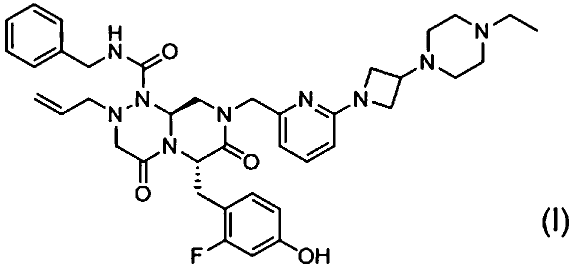 or a pharmaceutically acceptable salt thereof.
[8] A combination of (6S,9aS)-N-benzyl-8-({6-[3-(4-ethylpiperazin-1-yl)azetidin-1-yl]pyridin-2-yl}methyl)-6-(2-fluoro-4-hydroxybenzyl)-4,7-dioxo-2-(prop-2-en-1-yl)hexahydro-2H-pyrazino[2,1-c][1,2,4]triazine-1(6H)-carboxamide represented by the following formula (I) 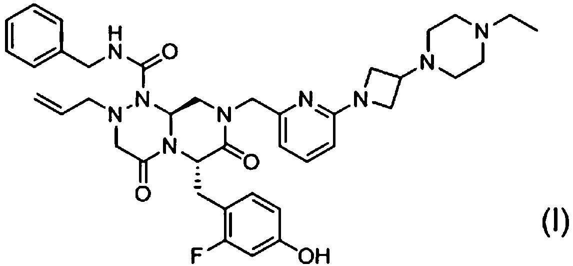 or a pharmaceutically acceptable salt thereof and a MEK inhibitor for use in the treatment of a tumor.
[9] A pharmaceutical composition for treating a tumor or a therapeutic agent for a tumor, comprising (6S,9aS)-N-benzyl-8-({6-[3-(4-ethylpiperazin-1-yl)azetidin-1-yl]pyridin-2-yl}methyl)-6-(2-fluoro-4-hydroxybenzyl)-4,7-dioxo-2-(prop-2-en-1-yl)hexahydro-2H-pyrazino[2,1-c][1,2,4]triazine-1(6H)-carboxamide represented by the following formula (I) 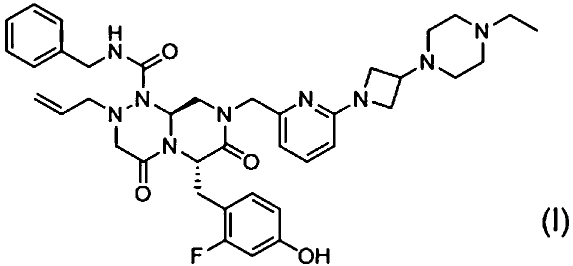 or a pharmaceutically acceptable salt thereof,
   wherein the composition or the agent is administered in combination with a MEK inhibitor and a RAS inhibitor.
[10] A pharmaceutical composition for treating a tumor or a therapeutic agent for a tumor, comprising a MEK inhibitor, wherein the composition or the agent is administered in combination with (6S,9aS)-N-benzyl-8-({6-[3-(4-ethylpiperazin-1-yl)azetidin-1-yl]pyridin-2-yl}methyl)-6-(2-fluoro-4-hydroxybenzyl)-4,7-dioxo-2-(prop-2-en-1-yl)hexahydro-2H-pyrazino[2,1-c][1,2,4]triazine-1(6H)-carboxamide represented by the following formula (I) 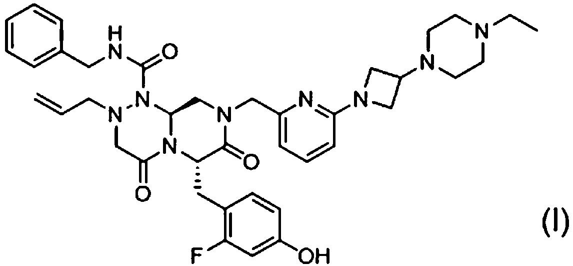 or a pharmaceutically acceptable salt thereof and a RAS inhibitor.
[11] A method of treating a tumor, wherein
   an effective amount of (6S,9aS)-N-benzyl-8-({6-[3-(4-ethylpiperazin-1-yl)azetidin-1-yl]pyridin-2-yl}methyl)-6-(2-fluoro-4-hydroxybenzyl)-4,7-dioxo-2-(prop-2-en-1-yl)hexahydro-2H-pyrazino[2,1-c][1,2,4]triazine-1(6H)-carboxamide represented by the following formula (I) 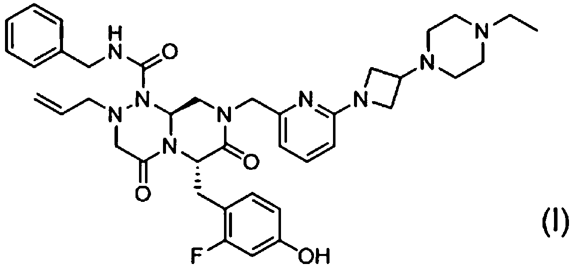
   or a pharmaceutically acceptable salt thereof, an effective amount of a MEK inhibitor, and an effective amount of a RAS inhibitor are administered to a patient in combination.
[12] Use of (6S,9aS)-N-benzyl-8-({6-[3-(4-ethylpiperazin-1-yl)azetidin-1-yl]pyridin-2-yl}methyl)-6-(2-fluoro-4-hydroxybenzyl)-4,7-dioxo-2-(prop-2-en-1-yl)hexahydro-2H-pyrazino[2,1-c][1,2,4]triazine-1(6H)-carboxamide represented by the following formula (I) 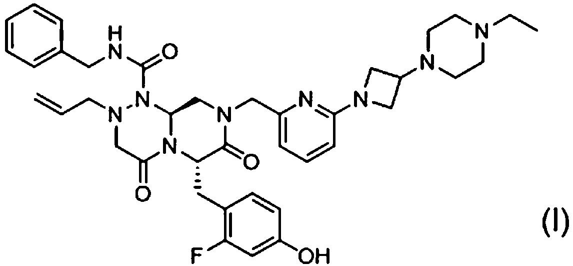 or a pharmaceutically acceptable salt thereof
   in the manufacture of a pharmaceutical composition for treating a tumor or a therapeutic agent for a tumor, wherein the composition or the agent is administered in combination with a MEK inhibitor and a RAS inhibitor.
[13] Use of a MEK inhibitor in the manufacture of a pharmaceutical composition for treating a tumor or a therapeutic agent for a tumor, wherein the composition or the agent is administered in combination with (6S,9aS)-N-benzyl-8-({6-[3-(4-ethylpiperazin-1-yl)azetidin-1-yl]pyridin-2-yl} methyl)-6-(2-fluoro-4-hydroxybenzyl)-4,7-dioxo-2-(prop-2-en-1-yl)hexahydro-2H-pyrazino[2,1-c][1,2,4]triazine-1(6H)-carboxamide represented by the following formula (I) 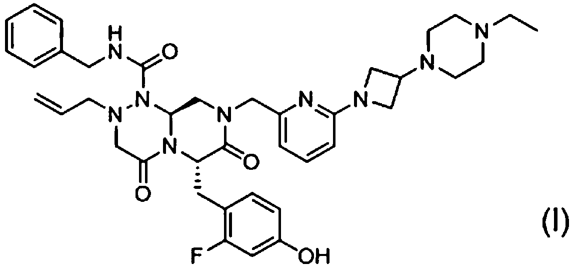 or a pharmaceutically acceptable salt thereof and a RAS inhibitor.
[14] (6S,9aS)-N-benzyl-8-({6-[3-(4-ethylpiperazin-1-yl)azetidin-1-yl]pyridin-2-yl}methyl)-6-(2-fluoro-4-hydroxybenzyl)-4,7-dioxo-2-(prop-2-en-1-yl)hexahydro-2H-pyrazino[2,1-c][1,2,4]triazine-1(6H)-carboxamide represented by the following formula (I) 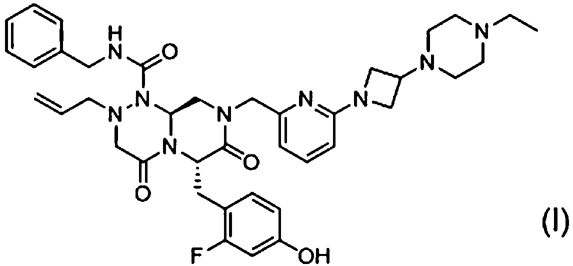 or a pharmaceutically acceptable salt thereof for use in the treatment of a tumor in combination with a MEK inhibitor and a RAS inhibitor.
[15] A MEK inhibitor for use in the treatment of a tumor in combination with (6S,9aS)-N-benzyl-8-({6-[3-(4-ethylpiperazin-1-yl)azetidin-1-yl]pyridin-2-yl}methyl)-6-(2-fluoro-4-hydroxybenzyl)-4,7-dioxo-2-(prop-2-en-1-yl)hexahydro-2H-pyrazino[2,1-c][1,2,4]triazine-1(6H)-carboxamide represented by the following formula (I) 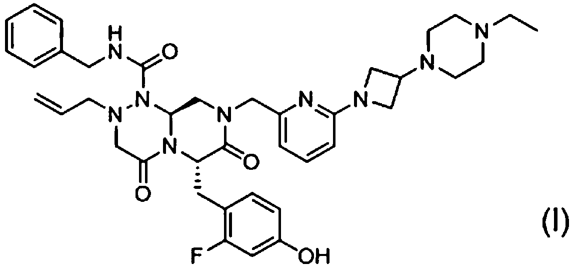 or a pharmaceutically acceptable salt thereof and a RAS inhibitor.
[16] A combination of (6S,9aS)-N-benzyl-8-({6-[3-(4-ethylpiperazin-1-yl)azetidin-1-yl]pyridin-2-yl}methyl)-6-(2-fluoro-4-hydroxybenzyl)-4,7-dioxo-2-(prop-2-en-1-yl)hexahydro-2H-pyrazino[2,1-c][1,2,4]triazine-1(6H)-carboxamide represented by the following formula (I) 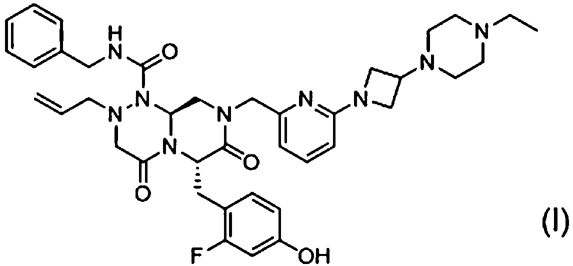 or a pharmaceutically acceptable salt thereof, a MEK inhibitor, and a RAS inhibitor t for use in the treatment of a tumor.
[17] A pharmaceutical composition for treating a tumor or a therapeutic agent for a tumor, comprising (6S,9aS)-N-benzyl-8-({6-[3-(4-ethylpiperazin-1-yl)azetidin-1-yl]pyridin-2-yl} methyl)-6-(2-fluoro-4-hydroxybenzyl)-4,7-dioxo-2-(prop-2-en-1-yl)hexahydro-2H-pyrazino[2,1-c][1,2,4]triazine-1(6H)-carboxamide represented by the following formula (I) 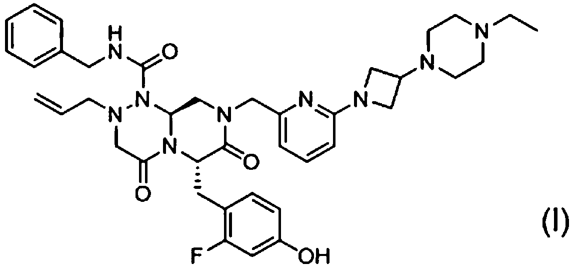 or a pharmaceutically acceptable salt thereof,
   wherein the composition or the agent is administered in combination with a MEK inhibitor and a BRAF inhibitor.
[18] A pharmaceutical composition for treating a tumor or a therapeutic agent for a tumor, comprising a MEK inhibitor,
   wherein the composition or the agent is administered in combination with (6S,9aS)-N-benzyl-8-({6-[3-(4-ethylpiperazin-1-yl)azetidin-1-yl]pyridin-2-yl}methyl)-6-(2-fluoro-4-hydroxybenzyl)-4,7-dioxo-2-(prop-2-en-1-yl)hexahydro-2H-pyrazino[2,1-c][1,2,4]triazine-1(6H)-carboxamide represented by the following formula (I) 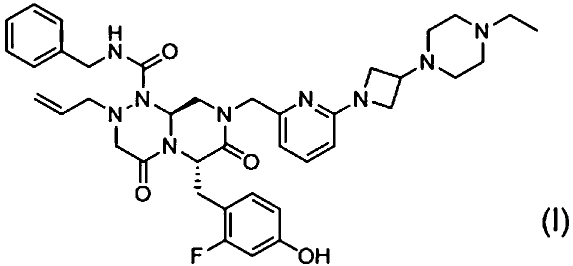 or a pharmaceutically acceptable salt thereof and a BRAF inhibitor.
[19] A method of treating a tumor, wherein
   an effective amount of (6S,9aS)-N-benzyl-8-({6-[3-(4-ethylpiperazin-1-yl)azetidin-1-yl]pyridin-2-yl}methyl)-6-(2-fluoro-4-hydroxybenzyl)-4,7-dioxo-2-(prop-2-en-1-yl)hexahydro-2H-pyrazino[2,1-c][1,2,4]triazine-1(6H)-carboxamide represented by the following formula (I) 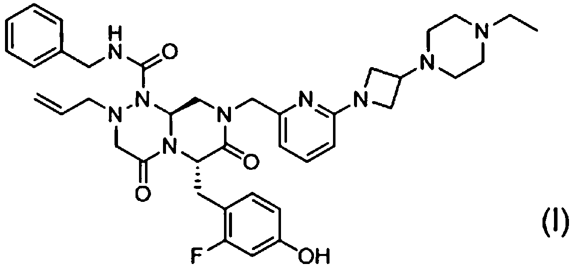
   or a pharmaceutically acceptable salt thereof, an effective amount of a MEK inhibitor, and an effective amount of a BRAF inhibitor are administered to a patient in combination.
[20] Use of (6S,9aS)-N-benzyl-8-({6-[3-(4-ethylpiperazin-1-yl)azetidin-1-yl]pyridin-2-yl} methyl)-6-(2-fluoro-4-hydroxybenzyl)-4,7-dioxo-2-(prop-2-en-1-yl)hexahydro-2H-pyrazino[2,1-c][1,2,4]triazine-1(6H)-carboxamide represented by the following formula (I) 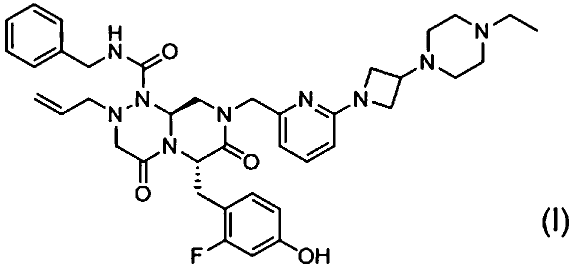
   or a pharmaceutically acceptable salt thereof
   in the manufacture of a pharmaceutical composition for treating a tumor or a therapeutic agent for a tumor,
   wherein the composition or the agent is administered in combination with a MEK inhibitor and a BRAF inhibitor.
[21] Use of a MEK inhibitor in the manufacture of a pharmaceutical composition for treating a tumor or a therapeutic agent for a tumor,
   wherein the composition or the agent is administered in combination with (6S,9aS)-N-benzyl-8-({6-[3-(4-ethylpiperazin-1-yl)azetidin-1-yl]pyridin-2-yl}methyl)-6-(2-fluoro-4-hydroxybenzyl)-4,7-dioxo-2-(prop-2-en-1-yl)hexahydro-2H-pyrazino[2,1-c][1,2,4]triazine-1(6H)-carboxamide represented by the following formula (I) 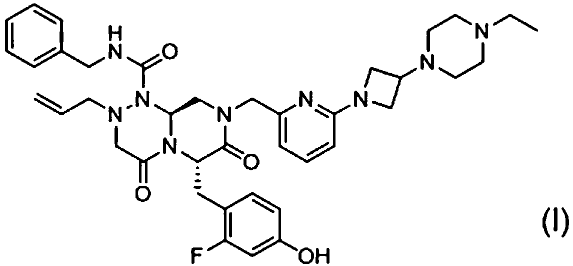 or a pharmaceutically acceptable salt thereof and a BRAF inhibitor.
[22] (6S,9aS)-N-benzyl-8-({6-[3-(4-ethylpiperazin-1-yl)azetidin-1-yl]pyridin-2-yl} methyl)-6-(2-fluoro-4-hydroxybenzyl)-4,7-dioxo-2-(prop-2-en-1-yl)hexahydro-2H-pyrazino[2,1-c][1,2,4]triazine-1(6H)-carboxamide represented by the following formula (I) 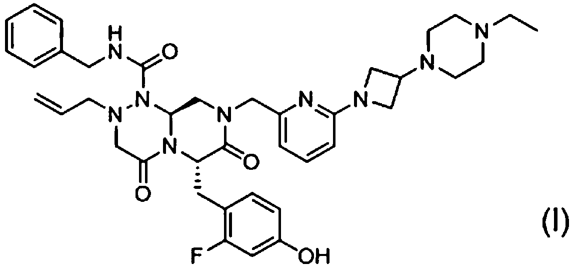 or a pharmaceutically acceptable salt thereof for use in the treatment of a tumor in combination with a MEK inhibitor and a BRAF inhibitor.
[23] A MEK inhibitor for use in the treatment of a tumor in combination with (6S,9aS)-N-benzyl-8-({6-[3-(4-ethylpiperazin-1-yl)azetidin-1-yl]pyridin-2-yl}methyl)-6-(2-fluoro-4-hydroxybenzyl)-4,7-dioxo-2-(prop-2-en-1-yl)hexahydro-2H-pyrazino[2,1-c][1,2,4]triazine-1(6H)-carboxamide represented by the following formula (I) 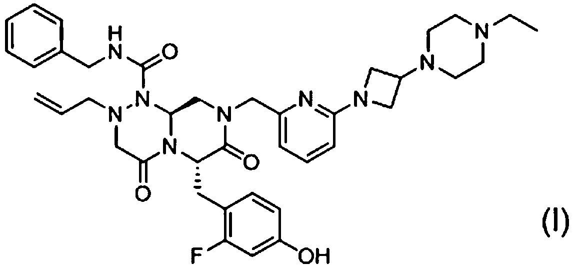 or a pharmaceutically acceptable salt thereof and a BRAF inhibitor.
[24] A combination of (6S,9aS)-N-benzyl-8-({6-[3-(4-ethylpiperazin-1-yl)azetidin-1-yl]pyridin-2-yl}methyl)-6-(2-fluoro-4-hydroxybenzyl)-4,7-dioxo-2-(prop-2-en-1-yl)hexahydro-2H-pyrazino[2,1-c][1,2,4]triazine-1(6H)-carboxamide represented by the following formula (I) 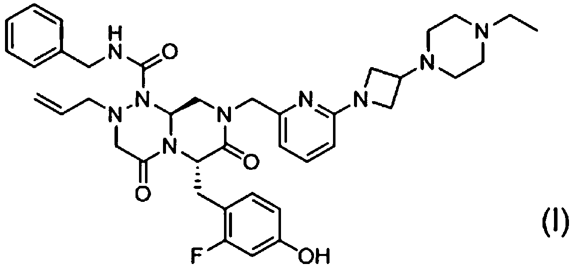 or a pharmaceutically acceptable salt thereof, a MEK inhibitor, and a BRAF inhibitor for use in the treatment of a tumor.
[25] The pharmaceutical composition, the therapeutic agent, the method, the use, the combination for use, the compound for use, or the MEK inhibitor for use according to any one of [1] to [24], wherein (6S,9aS)-N-benzyl-8-({6-[3-(4-ethylpiperazin-1-yl)azetidin-1-yl]pyridin-2-yl}methyl)-6-(2-fluoro-4-hydroxybenzyl)-4,7-dioxo-2-(prop-2-en-1-yl)hexahydro-2H-pyrazino[2,1-c][1,2,4]triazine-1(6H)-carboxamide or a pharmaceutically acceptable salt thereof is (6S,9aS)-N-benzyl-8-({6-[3-(4-ethylpiperazin-1-yl)azetidin-1-yl]pyridin-2-yl}methyl)-6-(2-fluoro-4-hydroxybenzyl)-4,7-dioxo-2-(prop-2-en-1-yl)hexahydro-2H-pyrazino[2,1-c][1,2,4]triazine-1(6H)-carboxamide.
[26] The pharmaceutical composition, the therapeutic agent, the method, the use, the combination for use, the compound for use or a pharmaceutically acceptable salt thereof, or the MEK inhibitor for use according to any one of [1] to [25], wherein the MEK inhibitor is at least one selected from Trametinib, Binimetinib, Selumetinib, Cobimetinib, and pharmaceutically acceptable salts thereof; and solvates thereof.
[27] The pharmaceutical composition, the therapeutic agent, the method, the use, the combination for use, the compound for use or a pharmaceutically acceptable salt thereof, or the MEK inhibitor for use according to any one of [1] to [25], wherein the MEK inhibitor is selected from Trametinib, Binimetinib, Selumetinib, Cobimetinib, and pharmaceutically acceptable salts thereof; and solvates thereof.
[28] The pharmaceutical composition, the therapeutic agent, the method, the use, the combination for use, the compound for use or a pharmaceutically acceptable salt thereof, or the MEK inhibitor for use according to any one of [1] to [25], wherein the MEK inhibitor is selected from Trametinib, Binimetinib, and pharmaceutically acceptable salts thereof; and solvates thereof.
[29] The pharmaceutical composition, the therapeutic agent, the method, the use, the combination for use, the compound for use or a pharmaceutically acceptable salt thereof, or the MEK inhibitor for use according to any one of [1] to [25], wherein the MEK inhibitor is Trametinib or a pharmaceutically acceptable salt thereof, or a solvate thereof.
[30] The pharmaceutical composition, the therapeutic agent, the method, the use, the combination for use, the compound for use or a pharmaceutically acceptable salt thereof, or the MEK inhibitor for use according to any one of [1] to [25], wherein the MEK inhibitor is a Trametinib-dimethyl sulfoxide.
[31] The pharmaceutical composition, the therapeutic agent, the method, the use, the combination for use, the compound for use or a pharmaceutically acceptable salt thereof, or the MEK inhibitor for use according to any one of [9] to [16] and [25] to [30], wherein the RAS inhibitor is a KRAS inhibitor.
[32] The pharmaceutical composition, the therapeutic agent, the method, the use, the combination for use, the compound for use or a pharmaceutically acceptable salt thereof, or the MEK inhibitor for use according to [31], wherein the KRAS inhibitor is at least one selected from Sotorasib, Adagrasib, MRTX1133, BI-3406, BI 1701963, RG6330, LY3537982, JDQ443, RMC-6291, RMC-6236, LUNA18, ARS-1620, JNJ-74699157, GDC-6036, iExosomes, mRNA-5671, JAB-21822, BI 1823911, MK-1084, ELI-002, SDGR5, JAB-22000, ASP3082, G12Si-1, and pharmaceutically acceptable salts thereof; and solvates thereof.
[33] The pharmaceutical composition, the therapeutic agent, the method, the use, the combination for use, the compound for use or a pharmaceutically acceptable salt thereof, or the MEK inhibitor for use according to [31], wherein the KRAS inhibitor is selected from Sotorasib, Adagrasib, MRTX1133, BI-3406, BI 1701963, RG6330, LY3537982, JDQ443, RMC-6291, RMC-6236, LUNA18, ARS-1620, JNJ-74699157, GDC-6036, iExosomes, mRNA-5671, JAB-21822, BI 1823911, MK-1084, ELI-002, SDGR5, JAB-22000, ASP3082, G12Si-1, and pharmaceutically acceptable salts thereof; and solvates thereof.
[34] The pharmaceutical composition, the therapeutic agent, the method, the use, the combination for use, the compound for use or a pharmaceutically acceptable salt thereof, or the MEK inhibitor for use according to [31], wherein the KRAS inhibitor is selected from Sotorasib, Adagrasib, BI 1701963, and pharmaceutically acceptable salts thereof; and solvates thereof.
[35] The pharmaceutical composition, the therapeutic agent, the method, the use, the combination for use, the compound for use or a pharmaceutically acceptable salt thereof, or the MEK inhibitor for use according to [31], wherein the KRAS inhibitor is a KRAS G12C inhibitor.
[36] The pharmaceutical composition, the therapeutic agent, the method, the use, the combination for use, the compound for use or a pharmaceutically acceptable salt thereof, or the MEK inhibitor for use according to [35], wherein the KRAS G12C inhibitor is selected from Sotorasib, Adagrasib, and pharmaceutically acceptable salts thereof; and solvates thereof.
[37] The pharmaceutical composition, the therapeutic agent, the method, the use, the combination for use, the compound for use or a pharmaceutically acceptable salt thereof, or the MEK inhibitor for use according to [35], wherein the KRAS G12C inhibitor is Sotorasib or a pharmaceutically acceptable salt thereof, or a solvate thereof.
[38] The pharmaceutical composition, the therapeutic agent, the method, the use, the combination for use, the compound for use or a pharmaceutically acceptable salt thereof, or the MEK inhibitor for use according to [35], wherein the KRAS G12C inhibitor is Adagrasib or a pharmaceutically acceptable salt thereof, or a solvate thereof.
[39] The pharmaceutical composition, the therapeutic agent, the method, the use, the combination for use, the compound for use or a pharmaceutically acceptable salt thereof, or the MEK inhibitor for use according to [35], wherein the MEK inhibitor is a Trametinib-dimethyl sulfoxide, and the KRAS G12C inhibitor is Sotorasib.
[40] The pharmaceutical composition, the therapeutic agent, the method, the use, the combination for use, the compound for use or a pharmaceutically acceptable salt thereof, or the MEK inhibitor for use according to [35], wherein the MEK inhibitor is a Trametinib-dimethyl sulfoxide, and the KRAS G12C inhibitor is Adagrasib.
[41] The pharmaceutical composition, the therapeutic agent, the method, the use, the combination for use, the compound for use or a pharmaceutically acceptable salt thereof, or the MEK inhibitor for use according to any one of [17] to [30], wherein the BRAF inhibitor is at least one selected from Dabrafenib, Encorafenib, Vemurafenib, and pharmaceutically acceptable salts thereof; and solvates thereof.
[42] The pharmaceutical composition, the therapeutic agent, the method, the use, the combination for use, the compound for use or a pharmaceutically acceptable salt thereof, or the MEK inhibitor for use according to any one of [17] to [30], wherein the BRAF inhibitor is selected from Dabrafenib, Encorafenib, Vemurafenib, and pharmaceutically acceptable salts thereof; and solvates thereof.
[43] The pharmaceutical composition, the therapeutic agent, the method, the use, the combination for use, the compound for use or a pharmaceutically acceptable salt thereof, or the MEK inhibitor for use according to any one of [17] to [30], wherein the BRAF inhibitor is Dabrafenib or a pharmaceutically acceptable salt thereof, or a solvate thereof.
[44] The pharmaceutical composition, the therapeutic agent, the method, the use, the combination for use, the compound for use or a pharmaceutically acceptable salt thereof, or the MEK inhibitor for use according to any one of [17] to [30], wherein the BRAF inhibitor is a Dabrafenib mesylate or Encorafenib.
[45] The pharmaceutical composition, the therapeutic agent, the method, the use, the combination for use, the compound for use or a pharmaceutically acceptable salt thereof, or the MEK inhibitor for use according to [44], wherein the MEK inhibitor is a Trametinib-dimethyl sulfoxide, and the BRAF inhibitor is a Dabrafenib mesylate.
[46] The pharmaceutical composition, the therapeutic agent, the method, the use, the combination for use, the compound for use or a pharmaceutically acceptable salt thereof, or the MEK inhibitor for use according to [44], wherein the MEK inhibitor is Binimetinib, and the BRAF inhibitor is Encorafenib.
[47] The pharmaceutical composition, the therapeutic agent, or the use according to any one of [1], [4], [9], [12], [17], [20], and [25] to [46], wherein the pharmaceutical composition or the therapeutic agent comprising (6S,9aS)-N-benzyl-8-({6-[3-(4-ethylpiperazin-1-yl)azetidin-1-yl]pyridin-2-yl} methyl)-6-(2-fluoro-4-hydroxybenzyl)-4,7-dioxo-2-(prop-2-en-1-yl)hexahydro-2H-pyrazino[2,1-c][1,2,4]triazine-1(6H)-carboxamide or a pharmaceutically acceptable salt thereof is for oral administration.
[48] The pharmaceutical composition, the therapeutic agent, the method, the use, the combination for use, the compound for use or a pharmaceutically acceptable salt thereof, or the MEK inhibitor for use according to any one of [2], [3], [5] to [8], [10], [11], [13] to [16], [18], [19], [21] to [24], and [25] to [46], wherein (6S,9aS)-N-benzyl-8-({6-[3-(4-ethylpiperazin-1-yl)azetidin-1-yl]pyridin-2-yl} methyl)-6-(2-fluoro-4-hydroxybenzyl)-4,7-dioxo-2-(prop-2-en-1-yl)hexahydro-2H-pyrazino[2,1-c][1,2,4]triazine-1(6H)-carboxamide or a pharmaceutically acceptable salt thereof is orally administered.
[49] The pharmaceutical composition, the therapeutic agent, the method, the use, the combination for use, the compound for use or a pharmaceutically acceptable salt thereof, or the MEK inhibitor for use according to any one of [1] to [48], wherein the tumor is at least one selected from non-small cell lung cancer, colorectal cancer, pancreatic cancer, mesothelioma, uterine cancer, ovarian cancer, bladder cancer, bile duct cancer, stomach cancer, breast cancer, small cell lung cancer, testicular cancer, small intestine cancer, appendix cancer, neuroendocrine tumor, melanoma, and head and neck cancer.
[50] The pharmaceutical composition, the therapeutic agent, the method, the use, the combination for use, the compound for use or a pharmaceutically acceptable salt thereof, or the MEK inhibitor for use according to any one of [1] to [8], [25] to [30], [47], and [48], wherein the tumor is at least one selected from non-small cell lung cancer, colorectal cancer, and melanoma.
[51] The pharmaceutical composition, the therapeutic agent, the method, the use, the combination for use, the compound for use or a pharmaceutically acceptable salt thereof, or the MEK inhibitor for use according to any one of [9] to [16], [25] to [40], [47], and [48], wherein the tumor is at least one selected from non-small cell lung cancer, colorectal cancer, and pancreatic cancer.
[52] The pharmaceutical composition, the therapeutic agent, the method, the use, the combination for use, the compound for use or a pharmaceutically acceptable salt thereof, or the MEK inhibitor for use according to any one of [17] to [30] and [41] to [48], wherein the tumor is at least one selected from colorectal cancer and melanoma.
[53] The pharmaceutical composition, the therapeutic agent, the method, the use, the combination for use, the compound for use or a pharmaceutically acceptable salt thereof, or the MEK inhibitor for use according to any one of [1] to [48], wherein the tumor is non-small cell lung cancer.
[54] The pharmaceutical composition, the therapeutic agent, the method, the use, the combination for use, the compound for use or a pharmaceutically acceptable salt thereof, or the MEK inhibitor for use according to any one of [1] to [48], wherein the tumor is colorectal cancer.
[55] The pharmaceutical composition, the therapeutic agent, the method, the use, the combination for use, the compound for use or a pharmaceutically acceptable salt thereof, or the MEK inhibitor to be used according to any one of [1] to [48], wherein the tumor is melanoma.

[A1] A pharmaceutical composition for treating a tumor or a therapeutic agent for a tumor, comprising (6S,9aS)-N-benzyl-8-({6-[3-(4-ethylpiperazin-1-yl)azetidin-1-yl]pyridin-2-yl} methyl)-6-(2-fluoro-4-hydroxybenzyl)-4,7-dioxo-2-(prop-2-en-1-yl)hexahydro-2H-pyrazino[2,1-c][1,2,4]triazine-1(6H)-carboxamide represented by the following formula (I) 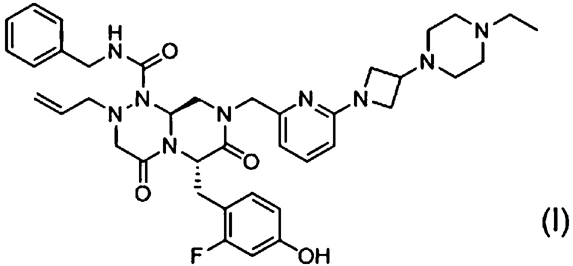 or a pharmaceutically acceptable salt thereof, wherein the composition or the agent is administered in combination with a BRAF inhibitor.
[A2] A pharmaceutical composition for treating a tumor or a therapeutic agent for a tumor, comprising a BRAF inhibitor, wherein the composition or the agent is administered in combination with (6S,9aS)-N-benzyl-8-({6-[3-(4-ethylpiperazin-1-yl)azetidin-1-yl]pyridin-2-yl} methyl)-6-(2-fluoro-4-hydroxybenzyl)-4,7-dioxo-2-(prop-2-en-1-yl)hexahydro-2H-pyrazino[2,1-c][1,2,4]triazine-1(6H)-carboxamide represented by the following formula (I) 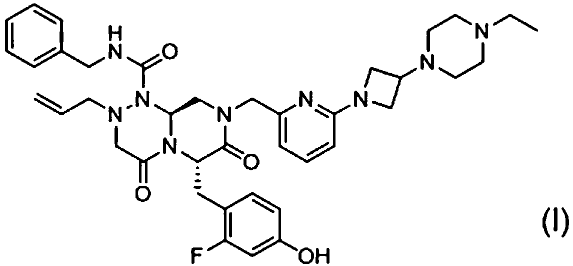 or a pharmaceutically acceptable salt thereof.
[A3] A method of treating a tumor, wherein
   an effective amount of (6S,9aS)-N-benzyl-8-({6-[3-(4-ethylpiperazin-1-yl)azetidin-1-yl]pyridin-2-yl} methyl)-6-(2-fluoro-4-hydroxybenzyl)-4,7-dioxo-2-(prop-2-en-1-yl)hexahydro-2H-pyrazino[2,1-c][1,2,4]triazine-1(6H)-carboxamide represented by the following formula (I) 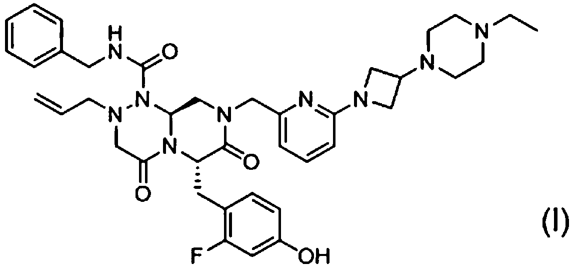
   or a pharmaceutically acceptable salt thereof and an effective amount of a BRAF inhibitor are administered to a patient in combination.
[A4] Use of (6S,9aS)-N-benzyl-8-({6-[3-(4-ethylpiperazin-1-yl)azetidin-1-yl]pyridin-2-yl}methyl)-6-(2-fluoro-4-hydroxybenzyl)-4,7-dioxo-2-(prop-2-en-1-yl)hexahydro-2H-pyrazino[2,1-c][1,2,4]triazine-1(6H)-carboxamide represented by the following formula (I) 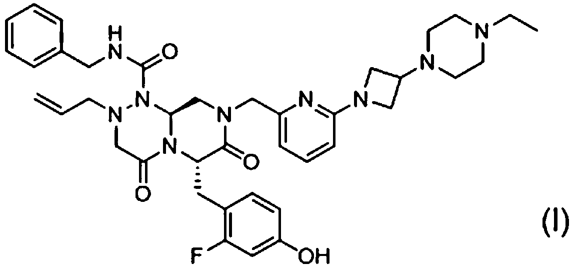
   or a pharmaceutically acceptable salt thereof
   in the manufacture of a pharmaceutical composition for treating a tumor or a therapeutic agent for a tumor,
   wherein the composition or the agent is administered in combination with a BRAF inhibitor.
[A5] Use of a BRAF inhibitor in the manufacture of a pharmaceutical composition for treating a tumor or a therapeutic agent for a tumor, wherein the composition or the agent is administered in combination with (6S,9aS)-N-benzyl-8-({6-[3-(4-ethylpiperazin-1-yl)azetidin-1-yl]pyridin-2-yl}methyl)-6-(2-fluoro-4-hydroxybenzyl)-4,7-dioxo-2-(prop-2-en-1-yl)hexahydro-2H-pyrazino[2,1-c][1,2,4]triazine-1(6H)-carboxamide represented by the following formula (I) 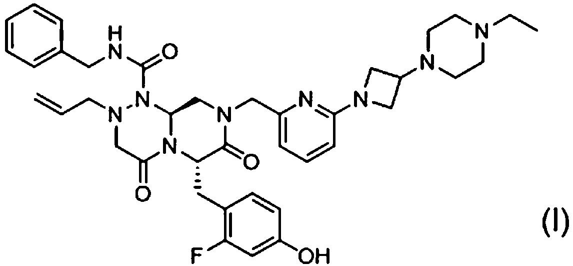 or a pharmaceutically acceptable salt thereof.
[A6] (6S,9aS)-N-benzyl-8-({6-[3-(4-ethylpiperazin-1-yl)azetidin-1-yl]pyridin-2-yl}methyl)-6-(2-fluoro-4-hydroxybenzyl)-4,7-dioxo-2-(prop-2-en-1-yl)hexahydro-2H-pyrazino[2,1-c][1,2,4]triazine-1(6H)-carboxamide represented by the following formula (I) 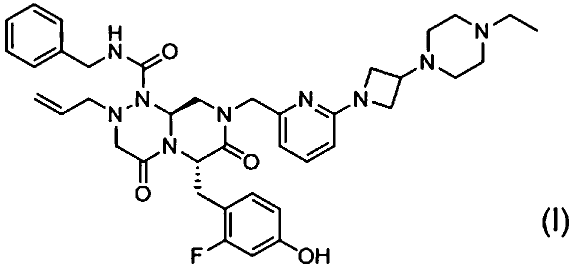 or a pharmaceutically acceptable salt thereof for use in the treatment of a tumor in combination with a BRAF inhibitor.
[A7] A BRAF inhibitor for use in the treatment of a tumor in combination with (6S,9aS)-N-benzyl-8-({6-[3-(4-ethylpiperazin-1-yl)azetidin-1-yl]pyridin-2-yl}methyl)-6-(2-fluoro-4-hydroxybenzyl)-4,7-dioxo-2-(prop-2-en-1-yl)hexahydro-2H-pyrazino[2,1-c][1,2,4]triazine-1(6H)-carboxamide represented by the following formula (I) 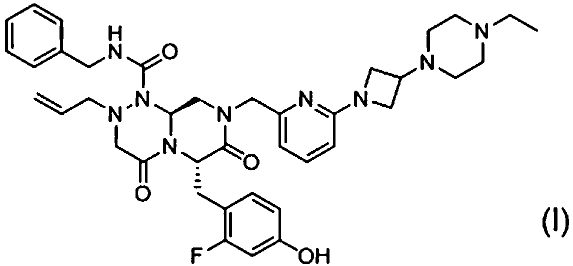 or a pharmaceutically acceptable salt thereof.
[A8] A combination of (6S,9aS)-N-benzyl-8-({6-[3-(4-ethylpiperazin-1-yl)azetidin-1-yl]pyridin-2-yl}methyl)-6-(2-fluoro-4-hydroxybenzyl)-4,7-dioxo-2-(prop-2-en-1-yl)hexahydro-2H-pyrazino[2,1-c][1,2,4]triazine-1(6H)-carboxamide represented by the following formula (I) 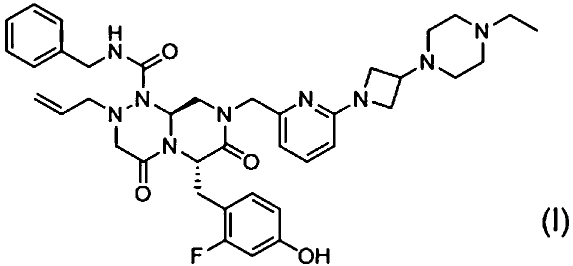 or a pharmaceutically acceptable salt thereof and a BRAF inhibitor for use in the treatment of a tumor.
[A9] The pharmaceutical composition, the therapeutic agent, the method, the use, the combination for use, the compound for use or a pharmaceutically acceptable salt thereof, or the BRAF inhibitor for use according to any one of [A1] to [A8], wherein (6S,9aS)-N-benzyl-8-({6-[3-(4-ethylpiperazin-1-yl)azetidin-1-yl]pyridin-2-yl}methyl)-6-(2-fluoro-4-hydroxybenzyl)-4,7-dioxo-2-(prop-2-en-1-yl)hexahydro-2H-pyrazino[2,1-c][1,2,4]triazine-1(6H)-carboxamide or a pharmaceutically acceptable salt thereof is (6S,9aS)-N-benzyl-8-({6-[3-(4-ethylpiperazin-1-yl)azetidin-1-yl]pyridin-2-yl} methyl)-6-(2-fluoro-4-hydroxybenzyl)-4,7-dioxo-2-(prop-2-en-1-yl)hexahydro-2H-pyrazino[2,1-c][1,2,4]triazine-1(6H)-carboxamide.
[A10] The pharmaceutical composition, the therapeutic agent, the method, the use, the combination for use, the compound for use or a pharmaceutically acceptable salt thereof, or the BRAF inhibitor for use according to any one of [A1] to [A9], wherein the BRAF inhibitor is selected from Dabrafenib, Encorafenib, and pharmaceutically acceptable salts thereof; and solvates thereof.
[A11] The pharmaceutical composition, the therapeutic agent, the method, the use, the combination for use, the compound for use or a pharmaceutically acceptable salt thereof, or the BRAF inhibitor for use according to any one of [A1] to [A9], wherein the BRAF inhibitor is selected from a Dabrafenib mesylate and Encorafenib.
[A12] The pharmaceutical composition, the therapeutic agent, or the use according to any one of [A1], [A4], and [A9] to [A11], wherein the pharmaceutical composition or the therapeutic agent comprising (6S,9aS)-N-benzyl-8-({6-[3-(4-ethylpiperazin-1-yl)azetidin-1-yl]pyridin-2-yl}methyl)-6-(2-fluoro-4-hydroxybenzyl)-4,7-dioxo-2-(prop-2-en-1-yl)hexahydro-2H-pyrazino[2,1-c][1,2,4]triazine-1(6H)-carboxamide or a pharmaceutically acceptable salt thereof is for oral administration.
[A13] The pharmaceutical composition, the therapeutic agent, the method, the use, the combination for use, the compound for use or a pharmaceutically acceptable salt thereof, or the BRAF inhibitor for use according to any one of [A2], [A3], [A5] to [A7], and [A9] to [A11], wherein (6S,9aS)-N-benzyl-8-({6-[3-(4-ethylpiperazin-1-yl)azetidin-1-yl]pyridin-2-yl}methyl)-6-(2-fluoro-4-hydroxybenzyl)-4,7-dioxo-2-(prop-2-en-1-yl)hexahydro-2H-pyrazino[2,1-c][1,2,4]triazine-1(6H)-carboxamide or a pharmaceutically acceptable salt thereof is orally administered.
[A14] The pharmaceutical composition, the therapeutic agent, the method, the use, the compound for use or a pharmaceutically acceptable salt thereof, or the BRAF inhibitor for use according to any one of [A1] to [A13], wherein the tumor is at least one selected from non-small cell lung cancer, colorectal cancer, and melanoma.

### Advantageous Effects of Invention

According to the present invention, a novel pharmaceutical composition for treating a tumor which exhibits a high antitumor effect can be provided.

### Brief Description of Drawings

FIG. 1 is a figure showing an antitumor effect of combined administration of the compound represented by formula (I) (abbreviated as E7386 in the figure) and Trametinib in a transplantation model mouse with human colorectal cancer cell line Colo205 cells.
FIG. 2 is a figure showing an antitumor effect of combined administration of the compound represented by formula (I) (abbreviated as E7386 in the figure) and Trametinib in a transplantation model mouse with human colorectal cancer cell line Colo205 cells.
FIG. 3 is a figure showing an antitumor effect of combined administration of ICG001 and Trametinib in a transplantation model mouse with human colorectal cancer cell line Colo205 cells.
FIG. 4 is a figure showing an antitumor effect of combined administration of the compound represented by formula (I) (abbreviated as E7386 in the figure) and Trametinib or combined administration of ICG001 and Trametinib in a transplantation model mouse with human colorectal cancer cell line Colo205 cells.
FIG. 5 is a figure showing an antitumor effect of two-agent or three-agent combined administration of the compound represented by formula (I) (abbreviated as E7386 in the figure), Trametinib, and Adagrasib in a transplantation model mouse with human colorectal cancer cell line SW1463 cells.
FIG. 6 is a figure showing an antitumor effect of two-agent or three-agent combined administration of the compound represented by formula (I) (abbreviated as E7386 in the figure), Trametinib, and Adagrasib in a transplantation model mouse with human colorectal cancer cell line SW1463 cells.
FIG. 7 is a figure showing an antitumor effect of combined administration of the compound represented by formula (I) (abbreviated as E7386 in the figure) and Trametinib in a transplantation model mouse with human colorectal cancer cell line Colo205 cells.
FIG. 8 is a figure showing an antitumor effect of two-agent or three-agent combined administration of the compound represented by formula (I) (abbreviated as E7386 in the figure), Trametinib, and Dabrafenib in a transplantation model mouse with human colorectal cancer cell line Colo205 cells.
FIG. 9 is a figure showing an antitumor effect of two-agent or three-agent combined administration of the compound represented by formula (I) (abbreviated as E7386 in the figure), Trametinib, and Dabrafenib in a transplantation model mouse with human colorectal cancer cell line Colo205 cells.
FIG. 10 is a figure showing an antitumor effect of combined administration of the compound represented by formula (I) (abbreviated as E7386 in the figure) and Trametinib in a transplantation model mouse with human non-small cell lung cancer cell line NCI-H358 cells.
FIG. 11 is a figure showing an antitumor effect of two-agent or three-agent combined administration of the compound represented by formula (I) (abbreviated as E7386 in the figure), Trametinib, and Adagrasib in a transplantation model mouse with human non-small cell lung cancer cell line NCI-H358 cells.
FIG. 12 is a figure showing an antitumor effect of two-agent or three-agent combined administration of the compound represented by formula (I) (abbreviated as E7386 in the figure), Trametinib, and Adagrasib in a transplantation model mouse with human non-small cell lung cancer cell line NCI-H358 cells.
FIG. 13 is a figure showing an antitumor effect of combined administration of the compound represented by formula (I) (abbreviated as E7386 in the figure) and Trametinib in a transplantation model mouse with human melanoma cell line A375 cells.
FIG. 14 is a figure showing an antitumor effect of two-agent or three-agent combined administration of the compound represented by formula (I) (abbreviated as E7386 in the figure), Trametinib, and Dabrafenib in a transplantation model mouse with human melanoma cell line A375 cells.
FIG. 15 is a figure showing an antitumor effect of two-agent or three-agent combined administration of the compound represented by formula (I) (abbreviated as E7386 in the figure), Trametinib, and Dabrafenib in a transplantation model mouse with human melanoma cell line A375 cells.

### Description of Embodiments

Hereinafter, embodiments of the present disclosure will be described. The following embodiments are examples for the present disclosure, and are not intended to limit the present disclosure only to the embodiments. The present disclosure can be performed in various forms without departing from the gist thereof. Note that the literatures, publications, patent publications, and other patent literatures cited in the present specification are incorporated herein by reference.

In the present disclosure, a "pharmaceutically acceptable salt" is not particularly limited provided that it forms a salt with a compound, and examples thereof include an inorganic acid salt, an organic acid salt, an inorganic base salt, an organic base salt, and an acidic or basic amino acid salt.

Examples of the inorganic acid salt include a hydrochloride, a hydrobromide, a sulfate, a nitrate, and a phosphate, and examples of the organic acid salt include a carboxylate such as an acetate, a succinate, a fumarate, a maleate, a tartrate, a citrate, a lactate, a stearate, a benzoate, or a mandelate, and a sulfonate such as a methanesulfonate, an ethanesulfonate, a p-toluenesulfonate, or a benzenesulfonate.

Examples of the inorganic base salt include an alkali metal salt such as a sodium salt or a potassium salt, an alkaline earth metal salt such as a calcium salt or a magnesium salt, an aluminum salt, and an ammonium salt, and examples of the organic base salt include a diethylamine salt, a diethanolamine salt, a meglumine salt, and an N,N'-dibenzylethylenediamine salt.

Examples of the acidic amino acid salt include an aspartic acid salt and a glutamic acid salt, and examples of the basic amino acid salt include an arginine salt, a lysine salt, and an ornithine salt.

In the present disclosure, a solvate refers to a solid formed by a compound or a pharmaceutically acceptable salt thereof and a solvent molecule together, and the solid may be a crystal. The solvate also includes a hydrate formed of a compound or a pharmaceutically acceptable salt thereof and a water molecule. A solvent molecule contained in the solvate is not particularly limited provided that it forms a solvate with a compound or a pharmaceutically acceptable salt thereof, and examples thereof include: a ketone-based solvent such as acetone, 2-butanone, or cyclohexanone; an ester-based solvent such as methyl acetate or ethyl acetate; an ether-based solvent such as 1,2-dimethoxyethane or t-butyl methyl ether; an alcohol-based solvent such as methanol, ethanol, 1-propanol, or isopropanol; and a polar solvent such as N-methyl-2-pyrrolidone, N N-dimethylformamide, or dimethyl sulfoxide. The number of solvent molecules with respect to a compound or a pharmaceutically acceptable salt thereof is not particularly limited, and may be, for example, one molecule or two molecules.

A compound represented by formula (I) is (6S,9aS)-N-benzyl-8-({6-[3-(4-ethylpiperazin-1-yl)azetidin-1-yl]pyridin-2-yl}methyl)-6-(2-fluoro-4-hydroxybenzyl)-4,7-dioxo-2-(prop-2-en-1-yl)hexahydro-2H-pyrazino[2,1-c][1,2,4]triazine-1(6H)-carboxamide. The compound represented by formula (I) is also referred to as "(6S,9aS)-N-benzyl-8-((6-(3-(4-ethylpiperazin-1-yl)azetidine-1-yl)pyridin-2-yl)methyl)-6-((2-fluoro-4-hydroxyphenyl)methyl)-4,7-dioxo-2-(prop-2-en-1-yl)-octahydro-1H-pyrazino[2,1-c][1,2,4]triazine-1-carboxamide" or "E7386".

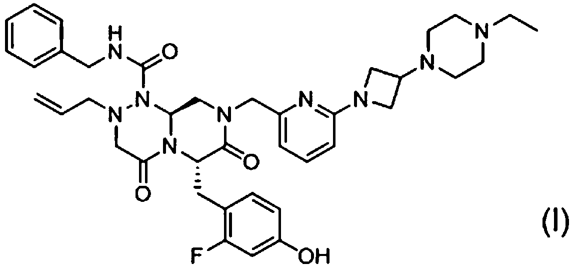

The compound represented by formula (I) can be manufactured by a method described in US Patent No. 9174998 or US Patent No. 10259817, and has pharmacological activity including antitumor activity.

In the present disclosure, "compound represented by formula (I) or a pharmaceutically acceptable salt thereof" may be an anhydride or a solvate such as a hydrate, and is, as an embodiment, for example, the compound represented by formula (I), that is, (6S,9aS)-N-benzyl-8-({6-[3-(4-ethylpiperazin-1-yl)azetidin-1-yl]pyridin-2-yl} methyl)-6-(2-fluoro-4-hydroxybenzyl)-4,7-dioxo-2-(prop-2-en-1-yl)hexahydro-2H-pyrazino[2,1-c][1,2,4]triazine-1(6H)-carboxamide.

"Pharmaceutical composition" or "therapeutic agent" in the present disclosure is understood to mean a substance that exhibits a desired effect in tissues, animals, mammals, humans, or other subjects.

In the present disclosure, "treating" and its derivatives mean therapeutic therapy. With reference to a specific condition, the treating means (1) ameliorating the condition, or one or more biological signs of the condition, (2) interfering with (a) one or more points in a biological cascade leading to or causing the condition, or (b) one or more biological signs of the condition, (3) alleviating one or more symptoms, influences, or side effects associated with the condition, or one or more symptoms, influences, or side effects associated with the condition or treatment thereof, or (4) delaying progression of the condition, or one or more biological signs of the condition.

In the present disclosure, "effective amount" means an amount effective to elicit a desired biological or pharmaceutical response in a tissue system, an animal, or a human. In an embodiment, the effective amount means an amount that is effective in achieving a desired therapeutic result when administered at the necessary dosages and for the required periods of time (for example, improvement or prevention of symptoms or prolongation of survival). In an embodiment, the effective amount is an amount that does not exceed a maximum tolerated dose. When one ingredient exhibiting a desired therapeutic effect is administered in combination with another ingredient, in an embodiment, the effective amount is equivalent to an effective amount when the one ingredient is administered as a single agent, and in another embodiment, the effective amount is a dosage smaller than an effective amount when the one ingredient is administered as a single agent.

In the present disclosure, "patient" refers to a mammal, particularly a human, suffering from or suspected of suffering from a tumor. The mammal refers to a mammal such as a guinea pig, a mouse, a rat, a gerbil, a cat, a rabbit, a dog, a cow, a goat, a sheep, a horse, a monkey, a chimpanzee, or a human. In an embodiment, the patient is a human. The pharmaceutical composition, the therapeutic agent, or the method of the present disclosure is particularly useful for treating human patients with tumors.

The terms "a", "an", and "the", and similar terms used in the context of the present disclosure (especially in the context of the claims) are to be construed to cover both the singular and the plural, unless otherwise indicated herein or clearly contradicted by context.

The pharmaceutical composition or therapeutic agent according to the present disclosure can be formulated by, for example, a method described in Japanese Pharmacopoeia 18 (JP), United States Pharmacopoeia (USP), or European Pharmacopoeia (EP). The pharmaceutical composition or the therapeutic agent according to the present disclosure can, in an embodiment, further contain a pharmaceutically acceptable carrier. Examples of the pharmaceutically acceptable carrier include a liquid or solid filler, a diluent, an excipient, a manufacturing aid, and a solvent encapsulating material.

In the present disclosure, the compound represented by formula (I) or a pharmaceutically acceptable salt thereof, or a pharmaceutical composition thereof or a therapeutic agent thereof can be administered by injection, oral administration, nasal administration, transdermal administration, pulmonary administration, ophthalmic administration, or the like. Examples of the injection include an intravenous injection, a subcutaneous injection, an intradermal injection, an intraarterial injection, and an injection such as a local injection into a target cell or organ. Examples of a dosage form of the pharmaceutical composition or the therapeutic agent in a case of oral administration include a tablet, a powder, a granule, a syrup, a capsule, and an internal liquid preparation. Examples of a dosage form of the pharmaceutical composition or the therapeutic agent in a case of parenteral administration include an injection, a drop, an eye drop, an ointment, a suppository, a suspension, a cataplasm, a lotion, an aerosol, and a plaster, and in an embodiment, the dosage form is an injection or a drop.

In an embodiment of the present disclosure, the compound represented by formula (I) or a pharmaceutically acceptable salt thereof is orally administered to a patient. In another embodiment, a pharmaceutical composition or a therapeutic agent comprising the compound represented by formula (I) or a pharmaceutically acceptable salt thereof is for oral administration.

In the present disclosure, an administration form of two or three ingredients selected from the compound represented by formula (I) or a pharmaceutically acceptable salt thereof, a MEK inhibitor, a RAS inhibitor, and a BRAF inhibitor and administered in combination is not particularly limited. For example, the two or three ingredients administered in combination are administered simultaneously, separately, sequentially, or at time intervals. Here, "simultaneously" means that the ingredients are administered within the same period or exactly simultaneously. "Separately" means that the ingredients are administered at different dosing intervals or frequencies. "Sequentially" means that the ingredients are administered in any order within a period. "At time intervals" means that each ingredient is administered at intervals for each administration thereof. Examples of a route of administration of each ingredient include injection, oral administration, nasal administration, transdermal administration, pulmonary administration, and ophthalmic administration. A case where two or three ingredients are administered in combination includes a case where the ingredients are administered as one preparation and a case where the ingredients are administered as two or more separate preparations.

A dosage of the compound represented by formula (I) or a pharmaceutically acceptable salt thereof at the time of administration as a single agent significantly varies depending on the type of disease to be targeted, the age, sex, and body weight of a patient, the degree of symptoms, and the like. The dosage of the compound represented by formula (I) or a pharmaceutically acceptable salt thereof is not particularly limited, but is usually 0.1 to 5000 mg, 0.5 to 3000 mg, or 1.0 to 1000 mg, and in an embodiment, 100 mg to 300 mg per day as the compound represented by formula (I) when the compound represented by formula (I) or a pharmaceutically acceptable salt thereof is orally administered to an adult (body weight: 60 kg) or a child. The compound represented by formula (I) or a pharmaceutically acceptable salt thereof can be usually administered at this dosage once per one or more days or in 2 to 6 portions per day. The dosage and dosage regimen can be changed when one or more additional chemotherapeutic agents are used. The dosage regimen can be determined by a physician treating a particular patient. As an embodiment, for example, the compound represented by formula (I) is orally administered to a human subject at a dosage of 80 mg to 120 mg per dose, twice daily. A specific dosage, administration route, administration frequency, administration cycle, and the like of the compound represented by formula (I) or a pharmaceutically acceptable salt thereof are appropriately determined in consideration of the type of disease to be targeted, the age, sex, and body weight of a patient, the degree of symptoms, other agents to be administered in combination, and the like.

The MEK inhibitor in the present disclosure is a substance that inhibits an action of phosphorylating enzyme MEK (mitogen-activated protein (MAP) kinase/extracellular signal-regulated kinase (ERK)) present in a Raf/MEK/ERK signaling pathway (MAPK pathway) to block the MAPK pathway.

In the present disclosure, examples of the MEK inhibitor include Trametinib, Selumetinib, Binimetinib, Cobimetinib, and pharmaceutically acceptable salts thereof; and solvates thereof.

Trametinib (Trametinib: GSK1120212, also referred to as JTP-74057) has the following structure: 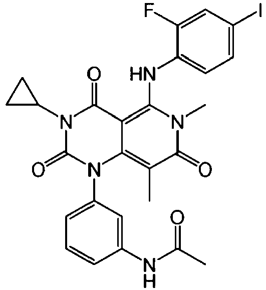 and means a compound whose chemical name is N-(3-(3-cyclopropyl-5-((2-fluoro-4-iodophenyl)amino)-6,8-dimethyl-2,4,7-trioxo-3,4,6,7-tetrahydropyrido[4,3-d]pyrimidine-1(2H)-yl)phenyl)acetamide. Trametinib is generally administered as a dimethyl sulfoxide solvate having the following structure:

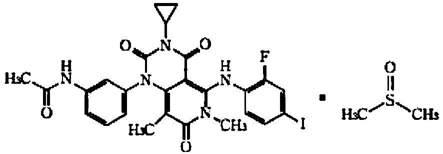

Trametinib (GSK1120212) is disclosed as Example 4-1 in U.S. Patent No. 7378423. A method for manufacturing Trametinib is described in Column 177, line 1 to Column 213, line 14, which is incorporated herein by reference.

A dosage of Trametinib or a pharmaceutically acceptable salt thereof, or a solvate thereof at the time of administration as a single agent significantly varies depending on the type of disease to be targeted, the age, sex, and body weight of a patient, the degree of symptoms, and the like. The dosage of Trametinib or a pharmaceutically acceptable salt thereof, or a solvate thereof is not particularly limited, but is usually 0.1 to 10 mg, in an embodiment, 0.5 mg to 2 mg, and in another embodiment, 2 mg, 1.5 mg, 1 mg, or 0.5 mg per day as Trametinib when Trametinib or a pharmaceutically acceptable salt thereof, or a solvate thereof is orally administered to an adult (body weight: 60 kg) or a child. Trametinib or a pharmaceutically acceptable salt thereof, or a solvate thereof can be usually administered at this dosage once a day on an empty stomach, for example, one or more hours before or two or more hours after a meal. The dosage and dosage regimen can be changed when one or more additional chemotherapeutic agents are used. The dosage regimen can be determined by a physician treating a particular patient. As an embodiment, for example, Trametinib is orally administered to a human subject at a dosage of 0.5 mg to 2 mg per dose, once daily. A specific dosage, administration route, administration frequency, administration cycle, and the like of Trametinib or a pharmaceutically acceptable salt thereof, or a solvate thereof are appropriately determined in consideration of the type of disease to be targeted, the age, sex, and body weight of a patient, the degree of symptoms, other agents to be administered in combination, and the like.

Selumetinib has the following structure: 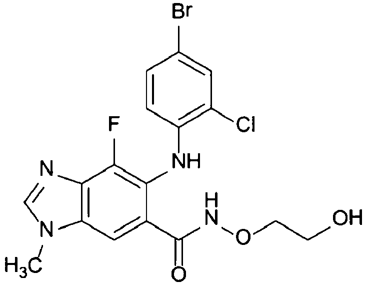 and means a compound whose chemical name is 5-[(4-Bromo-2-chlorophenyl)amino]-4-fluoro-N-(2-hydroxyethoxy)-1-methyl-1H-benzimidazole-6-carboxamide. Selumetinib is generally administered as a sulfate. Selumetinib is disclosed in Example 10 in WO 03/077914 A. A method for manufacturing Selumetinib is also described in Example 10, which is incorporated herein by reference.

Binimetinib has the following structure: 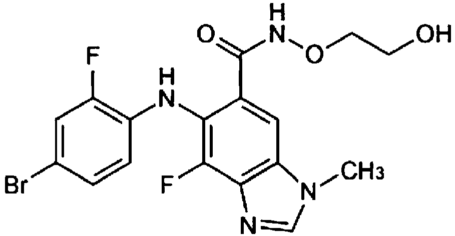 and means a compound whose chemical name is 5-[(4-Bromo-2-fluorophenyl)amino]-4-fluoro-N-(2-hydroxyethoxy)-1-methyl-1H-benzimidazole-6-carboxamide. Binimetinib is disclosed as 29111 of Example 18 in U.S. Patent No. 7777050. A method for manufacturing Binimetinib is also described in Example 18, which is incorporated herein by reference.

Cobimetinib has the following structure: 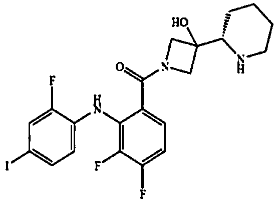 and a chemical name thereof is 1-[{3,4-difluoro-2-(2-fluoro-4-iodophenylamino)phenyl}carbonyl]-3-[(2S)-piperidin-2-yl]azetidin-3-ol. Cobimetinib is disclosed as Compound No. 28 in U.S. Patent No. 7803839. A general method for manufacturing Cobimetinib is described in Columns 221 to 237, and a method for manufacturing a compound thereof is described in Examples 22(b) in Columns 300 to 305, which are incorporated herein by reference.

In the present disclosure, examples of the MEK inhibitor include Trametinib, Selumetinib, Binimetinib, Cobimetinib, and pharmaceutically acceptable salts thereof; and solvates thereof. These may be used singly or in combination of two or more thereof. In an embodiment, the MEK inhibitor is selected from Trametinib, Selumetinib, Binimetinib, Cobimetinib, and pharmaceutically acceptable salts thereof; and solvates thereof. In another embodiment the MEK inhibitor is selected from Trametinib, Binimetinib, and pharmaceutically acceptable salts thereof; and solvates thereof,. In a particular embodiment, the MEK inhibitor is a Trametinib-dimethyl sulfoxide or Binimetinib.

The BRAF inhibitor in the present disclosure is a substance that targets, reduces, or inhibits activity of serine/threonine protein kinase BRAF. For example, the BRAF inhibitor also includes a substance that inhibits signal transduction downstream from BRAF, particularly a substance that specifically inhibits signal transduction caused by mutant BRAF having a gain of function in which serine threonine kinase activity is enhanced.

In the present disclosure, examples of the BRAF inhibitor include Dabrafenib, Encorafenib, Vemurafenib, and pharmaceutically acceptable salts thereof; and solvates thereof.

Dabrafenib has the following structure: 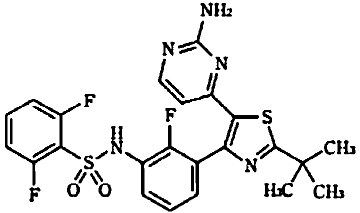 and a chemical name thereof is N-{3-[5-(2-amino-4-pyrimidinyl)-2-(1,1-dimethylethyl)-1,3-thiazole-4-yl]-2-fluorophenyl} - 2,6-difluorobenzenesulfonamide. A structure of this compound and a method for manufacturing this compound are disclosed as U.S. Patent No. 8415345, which is incorporated herein by reference.

Encorafenib has the following structure: 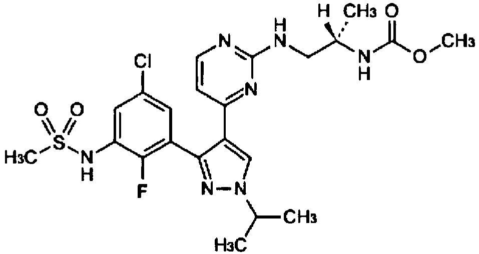 and a chemical name thereof is methyl N-{(2S)-1-[(4-{3-[5-chloro-2-fluoro-3-(methanesulfonamide)phenyl]-1-(propan-2-yl)-1H-pyrazol-4-yl}pyrimidin-2-yl)amino]propan-2-yl}carbamate. A structure of this compound is disclosed as Compound 32 in U.S. Patent No. 8501758, and a method for manufacturing this compound is also disclosed, which are incorporated herein by reference.

Vemurafenib (also referred to as ZELBORAF (registered trademark)) has the following structure: 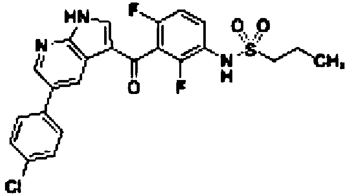 and a chemical name thereof is N-{3-[5-(4-chlorophenyl)-1H-pyrrolo[2,3-b]pyridine-3-carbonyl]-2,4-difluorophenyl}propane-1-sulfonamide. A structure of this compound is disclosed as P-1261 in U.S. Patent No. 8143271, and a method for manufacturing this compound is disclosed in U.S. Patent No. 8741920, which are incorporated herein by reference.

In the present disclosure, examples of an embodiment of the BRAF inhibitor include Dabrafenib, Encorafenib, and pharmaceutically acceptable salts thereof; and solvates thereof. An embodiment of the BRAF inhibitor is selected from Dabrafenib, Encorafenib, Vemurafenib, and pharmaceutically acceptable salts thereof; and solvates thereof. A specific embodiment of the BRAF inhibitor is selected from a Dabrafenib mesylate and Encorafenib.

A dosage of the BRAF inhibitor or a pharmaceutically acceptable salt thereof, or a solvate thereof is not particularly limited, but is usually 0.1 to 5000 mg, 0.5 to 3000 mg, or 1.0 to 1000 mg per day when the BRAF inhibitor or a pharmaceutically acceptable salt thereof, or a solvate thereof is orally administered to an adult (body weight: 60 kg) or a child. The BRAF inhibitor or a pharmaceutically acceptable salt thereof, or a solvate thereof can be usually administered at this dosage once per one or more days or in 2 to 6 portions per day. The dosage and dosage regimen can be changed when one or more additional chemotherapeutic agents are used. The dosage regimen can be determined by a physician treating a particular patient. When Dabrafenib is administered singly as the BRAF inhibitor, for example, Dabrafenib is orally administered at a dose selected from the group consisting of 300 mg, 200 mg, 150 mg, 125 mg, 100 mg, 75 mg, and 50 mg per day. A specific dosage, administration route, administration frequency, administration cycle, and the like of the BRAF inhibitor are appropriately determined in consideration of the type of disease to be targeted, the age, sex, and body weight of a patient, the degree of symptoms, other agents to be administered in combination, and the like.

The RAS inhibitor in the present disclosure may contain any compound or biomolecule that (a) binds to and inhibits a function of a biomolecule having RAS (for example, a protein or a nucleic acid), (b) binds to and degrades a biomolecule having RAS (for example, a protein or a nucleic acid), (c) suppresses expression of a biomolecule having RAS (for example, a protein or a nucleic acid), inhibits a GDP-GTP exchange reaction of RAS by inhibiting a protein-protein interaction between RAS and a guanine nucleotide exchange factor (for example, SOS1 or SOS2), or (e) functions as a vaccine to stimulate immunity against mutant RAS.

In the present disclosure, examples of an embodiment of the RAS inhibitor include Sotorasib (AMG 510), Adagrasib (MRTX849), MRTX1133, BI-3406, BI 1701963, RG6330, LY3537982, JDQ443, RMC-6291, RMC-6236, LUNA18, ARS-1620, JNJ-74699157, GDC-6036, iExosomes, D-1553, mRNA-5671, BI 2852, SCH-54292, TLN-4601, Salirasib, Deltarasin, JAB-21822, BI 1823911, MK-1084, ELI-002, SDGR5, JAB-22000, ASP3082, and G12Si-1.

Sotorasib (also referred to as AMG 510) has the following structure: 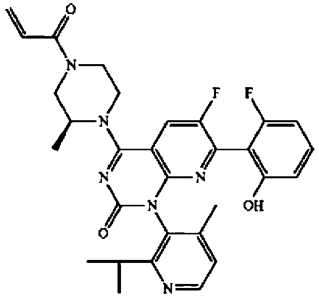 and means a compound whose chemical name is 6-fluoro-7-(2-fluoro-6-hydroxyphenyl)-1-(4-methyl-2-(2-propanyl)-3-pyridinyl)-4-((2S)-2-methyl-4-(2-propenoyl)-1-piperazinyl)pyrido[2,3-d]pyrimidin-2(1H)-one. A structural formula of Sotorasib (AMG 510) and a method for manufacturing Sotorasib are described in U.S. Patent No. 10519146, Column 411, line 10 to Column 415, line 19, which are incorporated herein by reference.

Adagrasib (also referred to as MRTX 849) has the following structure: 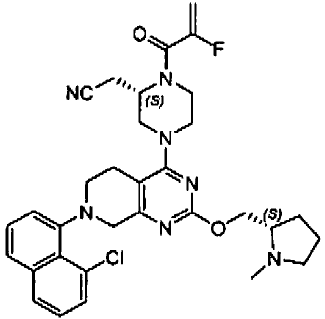 and means a compound whose chemical name is 2-[(2S)-4-[7-(8-chloro-1-naphthyl)-2-[[(2S)-1-methylpyrrolidin-2-yl]methoxy]-6,8-dihydro-5H-pyrido[3,4-d]pyrimidine-4-yl]-1-(2-fluoropropa-2-enoyl)piperazin-2-yl]acetonitrile. A structural formula of Adagrasib (MRTX849) and a method for manufacturing Adagrasib are described in U.S. Patent No. 10689377, Column 1286, line 32 to Column 1288, line 17, which are incorporated herein by reference.

MRTX1133 has the following structure: 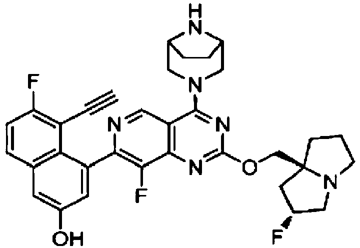 and means a compound whose chemical name is 4-(4-((1R,5S)-3,8-diazabicyclo[3.2.1]octan-3-yl)-8-fluoro-2-(((2R,7aS)-2-fluorohexahydro-1H-pyrrolidin-7a-yl)methoxy)pyrrodo[4,3-d]pyrimidin-7-yl)-5-ethynyl-6-fluoronaphthalen-2-ol. A structural formula of MRTX1133 and a method for manufacturing MRTX1133 are described in WO 2021041671 A.

A structural formula of BI-3406 and a method for manufacturing BI-3406 are described in U.S. Patent No. 10898487, which are incorporated herein by reference.

Pharmacological activity of BI 1701963 is reported in AACR Annual Meeting 2021 #CT210; 2021 Apr 10-15 and May 17-21 (Abstract https://doi.org/10.1158/1538-7445.AM2021-CT210).

RG6330 is the same compound as GDC-6036. A structural formula of RG6330 and a method for manufacturing RG6330 are described in U.S. Patent No. 11236068, which are incorporated herein by reference.

Pharmacological activity of LY3537982 is reported in AACR Annual Meeting 2021 #1259; Apr 10-15 and May 17-21 (Abstract https://doi.org/10.1158/1538-7445.AM2021-1259).

A structural formula of JDQ443 and a method for manufacturing JDQ443 are described in WO 2021124222 A.

A structural formula of ARS-1620 and a method for manufacturing ARS-1620 are described in U.S. Patent No. 10370386, which are incorporated herein by reference.

Pharmacological activity of iExosomes is reported in Nature volume 546, pages 498-503 (2017).

A structural formula of D-1553 and a method for manufacturing D-1553 are described in U.S. Patent No. 11091481, which are incorporated herein by reference.

A structural formula of BI 2852 and a method for manufacturing BI 2852 are described in WO 2020/173935 A.

A structural formula of SCH-54292 and a method for manufacturing SCH-54292 are reported in Bioorganic & Medicinal Chemistry, Volume 5, Issue 1, January 1997, Pages 125-133.

A structural formula of TLN-4601 and a method for manufacturing TLN-4601 are described in U.S. Patent No. 7101872, which are incorporated herein by reference.

A structural formula of Salirasib and a method for manufacturing Salirasib are described in U.S. Reissue Patent No. 39682, which are incorporated herein by reference.

A structural formula of Deltarasin and a method for manufacturing Deltarasin are described in U.S. Patent No. 9861623, which are incorporated herein by reference.

Pharmacological activity of JAB-21822 is reported in Journal of Clinical Oncology 40, no. 16_suppl (June 01, 2022) 3089-3089 (DOI: 10.1200/JCO. 2022.40.16_suppl. 3089).

Pharmacological activity of ELI-002 is reported in Journal of Clinical Oncology 40, no. 16_suppl (June 01, 2022) TPS2701-TPS2701 (DOI:10.1200/JCO.2022.40.16_suppl. TPS2701).

Pharmacological activity of G12Si-1 is reported in Nat Chem Biol 18, 1177-1183 (2022) (DOI: 10.1038/s41589 - 022-01065-9).

In the present disclosure, examples of the RAS inhibitor include Sotorasib (AMG 510), Adagrasib (MRTX849), MRTX1133, BI-3406, BI 1701963, RG6330, LY3537982, JDQ443, RMC-6291, RMC-6236, LUNA18, ARS-1620, JNJ-74699157, GDC-6036, iExosomes, D-1553, mRNA-5671, JAB-21822, BI 1823911, MK-1084, ELI-002, SDGR5, JAB-22000, BI 2852, SCH-54292, TLN-4601, Salirasib, Deltarasin, ASP3082, and G12Si-1, and these may be used singly or in combination of two or more thereof. In an embodiment, the RAS inhibitor is selected from Sotorasib, Adagrasib, MRTX1133, BI-3406, BI 1701963, RG6330, LY3537982, JDQ443, RMC-6291, RMC-6236, LUNA18, ARS-1620, JNJ-74699157, GDC-6036, iExosomes, mRNA-5671, JAB-21822, BI 1823911, MK-1084, ELI-002, SDGR5, JAB-22000, ASP3082, G12Si-1, and pharmaceutically acceptable salts thereof; and solvates thereof. In a specific embodiment, the RAS inhibitor is selected from Sotorasib (AMG 510), Adagrasib (MRTX849), BI 1701963, and pharmaceutically acceptable salts thereof; and solvates thereof. In another embodiment, the RAS inhibitor is Sotorasib. In another embodiment, the RAS inhibitor is Adagrasib.

In an embodiment of the present disclosure, the RAS inhibitor is a KRAS inhibitor, a NRAS inhibitor, or a HRAS inhibitor. In another embodiment of the present disclosure, the RAS inhibitor is a KRAS inhibitor.

In the present disclosure, the KRAS inhibitor means an inhibitor that (i) blocks a downstream signal caused by RAS by binding to KRAS or (ii) inhibits a protein-protein interaction between KRAS and a guanine nucleotide exchange factor (for example, SOS1 or SOS2). In the above (i), an inhibitor that binds to a GTP-binding mutant KRAS protein is a RAS-on inhibitor, and an inhibitor that binds to a GDP-binding mutant KRAS protein is a RAS-off inhibitor.

In an embodiment of the present disclosure, the KRAS inhibitor is selected from Sotorasib, Adagrasib, MRTX1133, BI-3406, BI 1701963, RG6330, LY3537982, JDQ443, RMC-6291, RMC-6236, LUNA18, ARS-1620, JNJ-74699157, GDC-6036, iExosomes, mRNA-5671, JAB-21822, BI 1823911, MK-1084, ELI-002, SDGR5, JAB-22000, ASP3082, G12Si-1, and pharmaceutically acceptable salts thereof; and solvates thereof.

In an embodiment of the present disclosure, the RAS-on inhibitor is selected from MRTX1133, RMC-6291, RMC-6236, and pharmaceutically acceptable salts thereof; and solvates thereof. In an embodiment of the present disclosure, the RAS-off inhibitor is selected from Sotorasib, Adagrasib, MRTX1133, RG6330, LY3537982, JDQ443, ARS-1620, JNJ-74699157, JAB-21822, BI 1823911, MK-1084, JAB-22000, G12Si-1, and pharmaceutically acceptable salts thereof; and solvates thereof.

Examples of a useful KRAS inhibitor include a KRAS G12C inhibitor, a KRAS G12D inhibitor, a KRAS G12S inhibitor, and a pan-KRAS inhibitor, and in an embodiment, the useful KRAS inhibitor is, for example, a KRAS G12C inhibitor. Here, the KRAS G12C inhibitor means an inhibitor that binds to a mutant of KRAS (for example, a protein or a nucleic acid) having a mutation in which the 12th Gly of KRAS is replaced with Cys and blocks a downstream signal caused by the mutant KRAS, and the KRAS G12D inhibitor means an inhibitor that binds to a mutant of KRAS (for example, a protein or a nucleic acid) having a mutation in which the 12th Gly of KRAS is replaced with Asp and blocks a downstream signal caused by the mutant KRAS. The KRAS G12S inhibitor means an inhibitor that binds to a mutant of KRAS (for example, a protein or a nucleic acid) having a mutation in which the 12th Gly of KRAS is replaced with Ser and blocks a downstream signal caused by the mutant KRAS. The pan-KRAS inhibitor means (a) an inhibitor that binds to KRAS (for example, a protein or a nucleic acid) and blocks a downstream signal caused by RAS regardless of presence or absence of mutation, a mutation position, and an amino acid after mutation, or (b) an inhibitor that blocks a downstream signal caused by RAS by binding to a guanine nucleotide exchange factor (for example, SOS1 or SOS2) of KRAS and inhibiting a protein-protein interaction between KRAS and the guanine nucleotide exchange factor.

In an embodiment, the KRAS G12C inhibitor is selected from Sotorasib (AMG 510), Adagrasib (MRTX849), RG6330, LY3537982, JDQ443, RMC-6291, JAB-21822, BI 1823911, MK-1084, and pharmaceutically acceptable salts thereof; and solvates thereof. In another embodiment, the KRAS G12C inhibitor is selected from Sotorasib (AMG 510), Adagrasib (MRTX849), and pharmaceutically acceptable salts thereof; and solvates thereof. In another embodiment, the KRAS G12C inhibitor is MK-1084. In another embodiment, the KRAS G12C inhibitor is Sotorasib or a pharmaceutically acceptable salt thereof, or a solvate thereof. In another embodiment, the KRAS G12C inhibitor is Adagrasib or a pharmaceutically acceptable salt thereof, or a solvate thereof. In a specific embodiment, the KRAS G12C inhibitor is Sotorasib (AMG 510). In another specific embodiment, the KRAS G12C inhibitor is Adagrasib (MRTX849). In an embodiment, the KRAS G12D inhibitor is selected from MRTX1133, JAB-22000, and pharmaceutically acceptable salts thereof; and solvates thereof. In an embodiment, the KRAS G12S inhibitor is G12Si-1 or a pharmaceutically acceptable salt thereof, or a solvate thereof. In an embodiment, the pan-KRAS inhibitor is selected from BI-3406, BI 1701963, LUNA18, SDGR5, RMC-6236, and pharmaceutically acceptable salts thereof; and solvates thereof.

A dosage of the RAS inhibitor or a pharmaceutically acceptable salt thereof, or a solvate thereof is not particularly limited, but is usually 0.1 to 5000 mg, 0.5 to 3000 mg, or 1.0 to 1000 mg per day when the RAS inhibitor or a pharmaceutically acceptable salt thereof, or a solvate thereof is orally administered to an adult (body weight: 60 kg) or a child. The RAS inhibitor or a pharmaceutically acceptable salt thereof, or a solvate thereof can be usually administered at this dosage once per one or more days or in 2 to 6 portions per day. The dosage and dosage regimen can be changed when one or more additional chemotherapeutic agents are used. The dosage regimen can be determined by a physician treating a particular patient. When Sotorasib is administered singly as the RAS inhibitor, for example, Sotorasib is orally administered at a dose selected from the group consisting of 960 mg, 480 mg, 240 mg, and 120 mg per day. When Adagrasib is administered singly as the RAS inhibitor, for example, Adagrasib is orally administered at a dose selected from the group consisting of 1200 mg, 800 mg, 600 mg, 400 mg, and 200 mg per day. A specific dosage, administration route, administration frequency, administration cycle, and the like of the RAS inhibitor are appropriately determined in consideration of the type of disease to be targeted, the age, sex, and body weight of a patient, the degree of symptoms, other agents to be administered in combination, and the like.

The MEK inhibitor, the RAS inhibitor, and the BRAF inhibitor according to the present disclosure can be each administered by injection, oral administration, nasal administration, transdermal administration, pulmonary administration, ophthalmic administration, or the like. Examples of the injection include an intravenous injection, a subcutaneous injection, an intradermal injection, an intraarterial injection, and an injection such as a local injection into a target cell or organ. Examples of a dosage form in a case of oral administration include a tablet, a powder, a granule, a syrup, a capsule, and an internal liquid preparation. Examples of a dosage form in a case of parenteral administration include an injection, a drop, an eye drop, an ointment, a suppository, a suspension, a cataplasm, a lotion, an aerosol, and a plaster, and in an embodiment, the dosage form is an injection or a drop. The MEK inhibitor, the RAS inhibitor, and the BRAF inhibitor according to the present disclosure can be formulated by, for example, a method described in Japanese Pharmacopoeia 18 (JP), United States Pharmacopoeia (USP), or European Pharmacopoeia (EP).

The tumor to be treated in the present disclosure is, for example, a solid cancer. Examples of the solid cancer among tumors to be treated include non-small cell lung cancer, colorectal cancer, pancreatic cancer, mesothelioma, uterine cancer (for example, cervical cancer or uterine body cancer), ovarian cancer, bladder cancer, bile duct cancer, stomach cancer, breast cancer, small cell lung cancer, testicular cancer, small intestine cancer, appendix cancer, neuroendocrine tumor, melanoma, and head and neck cancer. Examples of melanoma include BRAF V600 mutant melanoma. Examples of non-small cell lung cancer include KRAS mutation-positive non-small cell lung cancer. Colorectal cancer also includes KRAS mutation-positive colorectal cancer. In a pharmaceutical composition for treating tumors (or a tumor therapeutic agent) that contains the compound represented by formula (I) or a pharmaceutically acceptable salt thereof according to an embodiment and is administered in combination with a MEK inhibitor or a pharmaceutical composition for treating tumors (or a tumor therapeutic agent) that contains a MEK inhibitor and is administered in combination with the compound represented by formula (I) or a pharmaceutically acceptable salt thereof, the tumor to be treated may be non-small cell lung cancer, colorectal cancer, or melanoma, may be non-small cell lung cancer or colorectal cancer, may be non-small cell lung cancer or melanoma, or may be colorectal cancer or melanoma. In a pharmaceutical composition for treating tumors (or a tumor therapeutic agent) that contains the compound represented by formula (I) or a pharmaceutically acceptable salt thereof according to an embodiment and is administered in combination with a MEK inhibitor and a RAS inhibitor or a pharmaceutical composition for treating tumors (or a tumor therapeutic agent) that contains a MEK inhibitor and is administered in combination with the compound represented by formula (I) or a pharmaceutically acceptable salt thereof and a RAS inhibitor, the tumor to be treated may be non-small cell lung cancer, colorectal cancer, or pancreatic cancer, or may be non-small cell lung cancer. In a pharmaceutical composition for treating tumors (or a tumor therapeutic agent) that contains the compound represented by formula (I) or a pharmaceutically acceptable salt thereof according to an embodiment and is administered in combination with a MEK inhibitor and a BRAF inhibitor or a pharmaceutical composition for treating tumors (or a tumor therapeutic agent) that contains a MEK inhibitor and is administered in combination with the compound represented by formula (I) or a pharmaceutically acceptable salt thereof and a BRAF inhibitor, the tumor to be treated may be melanoma, or may be BRAF V600 mutant melanoma.

Examples of an embodiment of the present disclosure include a pharmaceutical composition for treating tumors (or a tumor therapeutic agent) that contains the compound represented by formula (I) and is administered in combination with a Trametinib-dimethyl sulfoxide and Sotorasib, and the tumor to be treated is colorectal cancer or non-small cell lung cancer.

Examples of an embodiment of the present disclosure include a pharmaceutical composition for treating tumors (or a tumor therapeutic agent) that contains the compound represented by formula (I) and is administered in combination with a Trametinib-dimethyl sulfoxide and Adagrasib, and the tumor to be treated is colorectal cancer or non-small cell lung cancer.

Examples of an embodiment of the present disclosure include a pharmaceutical composition for treating tumors (or a tumor therapeutic agent) that contains the compound represented by formula (I) and is administered in combination with a Trametinib-dimethyl sulfoxide, and the tumor to be treated is at least one selected from non-small cell lung cancer, colorectal cancer, and melanoma.

Examples of an embodiment of the present disclosure include a pharmaceutical composition for treating tumors (or a tumor therapeutic agent) that contains the compound represented by formula (I) and is administered in combination with a Trametinib-dimethyl sulfoxide and MK-1084, and the tumor to be treated is colorectal cancer or non-small cell lung cancer.

Examples of an embodiment of the present disclosure include a pharmaceutical composition for treating tumors (or a tumor therapeutic agent) that contains the compound represented by formula (I) and is administered in combination with a Trametinib-dimethyl sulfoxide and a Dabrafenib mesylate, and the tumor to be treated is colorectal cancer or melanoma, and in a specific embodiment, is BRAF V600 mutant melanoma.

Examples of an embodiment of the present disclosure include a pharmaceutical composition for treating tumors (or a tumor therapeutic agent) that contains the compound represented by formula (I) and is administered in combination with Binimetinib and Encorafenib, and the tumor to be treated is colorectal cancer or melanoma, and in a specific embodiment, is BRAF V600 mutant melanoma.

Examples of an embodiment of the present disclosure include a pharmaceutical composition for treating tumors (or a tumor therapeutic agent) that contains the compound represented by formula (I) and is administered in combination with a Dabrafenib mesylate or Encorafenib, and the tumor to be treated is at least one selected from non-small cell lung cancer, colorectal cancer, and melanoma.

Examples of an embodiment of the present disclosure include a pharmaceutical composition for treating tumors (or a tumor therapeutic agent) that contains the compound represented by formula (I) and is administered in combination with a Trametinib-dimethyl sulfoxide, and the tumor to be treated is at least one selected from non-small cell lung cancer, colorectal cancer, and melanoma.

In an embodiment of the present disclosure, there is provided a method of treating a tumor in which the compound represented by formula (I) or a pharmaceutically acceptable salt thereof and a MEK inhibitor are administered in combination, and in another embodiment, there is provided a method of treating a tumor in which a BRAF inhibitor is further administered in combination. In another embodiment, there is provided a method of treating a tumor in which a RAS inhibitor is further administered in combination. In an embodiment of the present disclosure, the compound represented by formula (I) or a pharmaceutically acceptable salt thereof is orally administered.

In addition, in an embodiment of the present disclosure, there is provided a method of treating a tumor in which the compound represented by formula (I) or a pharmaceutically acceptable salt thereof and a BRAF inhibitor are administered in combination.

An embodiment of the present disclosure is a method of treating a tumor in which the compound represented by formula (I), a Trametinib-dimethyl sulfoxide, and Sotorasib are combined and orally administered to a patient in an effective amount, and the tumor to be treated is colorectal cancer or non-small cell lung cancer.

An embodiment of the present disclosure is a method of treating a tumor in which the compound represented by formula (I), a Trametinib-dimethyl sulfoxide, and Adagrasib are combined and orally administered to a patient in an effective amount, and the tumor to be treated is colorectal cancer or non-small cell lung cancer.

An embodiment of the present disclosure is a method of treating a tumor in which the compound represented by formula (I) and a Trametinib-dimethyl sulfoxide are combined and orally administered to a patient in an effective amount, and the tumor to be treated is at least one selected from non-small cell lung cancer, colorectal cancer, and melanoma.

An embodiment of the present disclosure is a method of treating a tumor in which the compound represented by formula (I), a Trametinib-dimethyl sulfoxide, and MK-1084 are combined and orally administered to a patient in an effective amount, and the tumor to be treated is colorectal cancer or non-small cell lung cancer.

An embodiment of the present disclosure is a method of treating a tumor in which the compound represented by formula (I), a Trametinib-dimethyl sulfoxide, and a Dabrafenib mesylate are combined and orally administered to a patient in an effective amount, and the tumor to be treated is colorectal cancer or melanoma, and in a specific embodiment, is BRAF V600 mutant melanoma.

An embodiment of the present disclosure is a method of treating a tumor in which the compound represented by formula (I), Binimetinib, and Encorafenib are combined and orally administered to a patient in an effective amount, and the tumor to be treated is melanoma, and in a specific embodiment, is BRAF V600 mutant melanoma.

An embodiment of the present disclosure is a method of treating a tumor in which the compound represented by formula (I) and a Dabrafenib mesylate or Encorafenib are combined and orally administered to a patient in an effective amount, and the tumor to be treated is at least one selected from non-small cell lung cancer, colorectal cancer, and melanoma.

As used herein, the term "comprising" or "including" is intended to be open-ended and inclusive, and does not exclude additional, undescribed characteristics, unless the context clearly dictates otherwise, and includes the closed term "consisting of" or "consisting essentially of".

Unless defined differently, each of all terms (technical and scientific terms) used herein has the same meaning as broadly understood by those skilled in the art to which the present invention belongs. Each of the terms used herein is to be interpreted as having a meaning that is consistent with its meaning in this specification and related art, and is not to be interpreted in an idealized or overly formal sense, unless a different definition is explicitly stated.

### Examples

### [Example 1: Antitumor effect of combined administration of a MEK inhibitor and the compound represented by formula (I) or ICG001 in a transplantation model mouse with human colorectal cancer cell line Colo205 cells]

Human colorectal cancer-derived cell line Colo205 cells (supplied by ATCC) were suspended in Hanks' Balanced Salt Solution (HBSS) (manufactured by FUJIFILM Wako Chemicals) at 5 × 10⁷ cells/mL. 0.1 mL of the obtained cell suspension was transplanted subcutaneously to a right frank of 6-week nude mice (CAnN.Cg-Foxn1 nu/CrlCrlj, manufactured by Charles River Laboratories Japan, Inc.). Ten days after the transplantation, a major axis and a minor axis of a tumor generated in each mouse were measured with a digimatic caliper (manufactured by Mitutoyo Corp.), and the mice were divided into groups each including six mice such that an average tumor volume was approximately equal. The tumor volume was calculated according to the following formula.Tumor volume (mm3) = tumor major axis (mm) × tumor minor axis2 (mm2)/2

From ten days after the transplantation, the compound represented by formula (I) (12.5 mg/kg or 25 mg/kg, once a day, 14 days, orally administered), Trametinib (0.3 mg/kg, once a day, 14 days, orally administered), and ICG001 (100 mg/kg, once a day, 14 days, intraperitoneally administered) were administered singly or in combination to the mice in a single administration group or a combined administration group. Note that any one of the above agents was not administered to a control group.

Note that, at the time of administration, the compound represented by formula (I) was dissolved in 0.1 M hydrochloric acid at 1.25 mg/mL or 2.50 mg/mL, Trametinib (manufactured by Amadis Chemical) was dissolved in distilled water (pH 8.0) (manufactured by Otsuka Pharmaceutical Co., Ltd.) containing 0.5 w/v% of hydroxypropyl methyl cellulose (manufactured by FUJIFILM Wako Chemicals) and 0.2 w/v% of Tween80 (manufactured by Sigma) at 0.03 mg/mL, and ICG001 (manufactured by WuXi App Tec) was dissolved in physiological saline (manufactured by Otsuka Pharmaceutical Co., Ltd.) containing 3.5 w/v% of dimethylsulfoxide (manufactured by Wako Pure Chemical Industries, Ltd.) and 6.5 w/v% of Tween80 at 10 mg/mL.

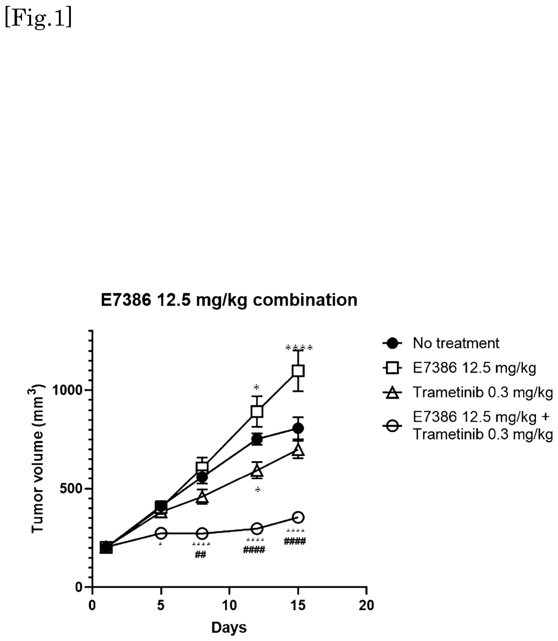

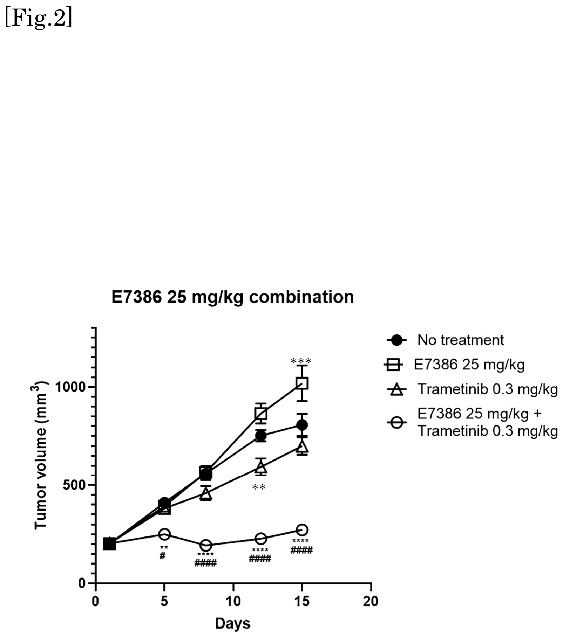

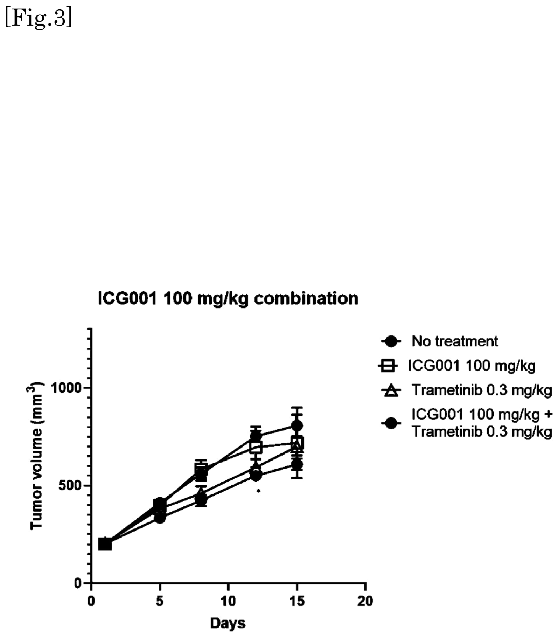

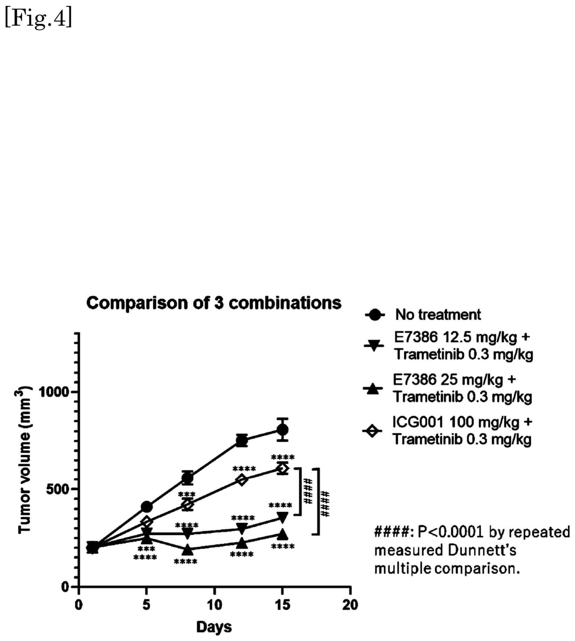

The administration start day was defined as the first day, and a major axis and a minor axis of a tumor generated in each mouse were measured on the fifth day, the eighth day, the twelfth day, and the fifteenth day, and a tumor volume was calculated by a similar method to the above-described method. FIG. 1 illustrates a transition of the tumor volume in a case where 12.5 mg/kg of the compound represented by formula (I) and 0.3 mg/kg of Trametinib were administered singly or in combination. FIG. 2 illustrates a transition of the tumor volume in a case where 25.0 mg/kg of the compound represented by formula (I) and 0.3 mg/kg of Trametinib were administered singly or in combination. FIG. 3 illustrates a transition of the tumor volume in a case where 100 mg/kg of ICG001 and 0.3 mg/kg of Trametinib were administered singly or in combination. FIG. 4 illustrates a transition of the tumor volume in a case where 12.5 mg/kg or 25.0 mg/kg of the compound represented by formula (I) or 100 mg/kg of ICG001 and 0.3 mg/kg of Trametinib were administered in combination. Note that, in the figures, the compound represented by formula (I) is described as E7386. In addition, a measurement result of the tumor volume of each group is indicated as a mean ± standard error (SEM).

As a result, the compound represented by formula (I) exhibited remarkable enhancement in antitumor effect by the combined use with Trametinib, and inhibited tumor proliferation in a statistically significant manner as compared with the case where the compound represented by formula (I) or Trametinib was administered singly (FIGS. 1 and 2). Such remarkable enhancement in antitumor effect was not observed in combined use of ICG001 and Trametinib (FIG. 3). In addition, the combined administration of the compound represented by formula (I) and Trametinib inhibited tumor proliferation in a statistically significant manner as compared with the combined administration of ICG001 and Trametinib (FIG. 4). *, **, ***, and **** in FIGS. 1 to 4 mean p < 0.05, p < 0.01, p < 0.001, and p< 0.0001 versus the control group (Repeated measures ANOVA followed by Dunnett's type multiple comparison), respectively. #, ##, ###, and #### in FIGS. 1 to 4 mean p < 0.05, p < 0.01, p < 0.001, and p< 0.0001 versus the single agent administration group; Repeated measures ANOVA followed by Dunnett's type multiple comparison), respectively.

### [Example 2: Antitumor effect of combined administration of a MEK inhibitor and the compound represented by formula (I), and combined administration of the MEK inhibitor, the compound represented by formula (I), and a KRAS inhibitor in a transplantation model mouse with human colorectal cancer cell line SW1463 cells]

Human colorectal cancer cell line SW1463 cells (supplied by ATCC) were suspended in Hanks' Balanced Salt Solution (HBSS) (manufactured by FUJIFILM Wako Chemicals) and Matrigel basement membrane matrix (manufactured by Corning International) (1 : 1 mixture) at 2 × 10⁸ cells/mL. 0.1 mL of the obtained cell suspension was transplanted subcutaneously to a right frank of 6-week nude mice (CAnN.Cg-Foxn1 nu/CrlCrlj, manufactured by Charles River Laboratories Japan, Inc.). Seven days after the transplantation, a major axis and a minor axis of a tumor generated in each mouse were measured with a digimatic caliper (manufactured by Mitutoyo Corp.), and the mice were divided into groups each including six mice such that an average tumor volume was approximately equal. The tumor volume was calculated according to the following formula.Tumor volume (mm3) = tumor major axis (mm) × tumor minor axis2 (mm2)/2

From nine days after the transplantation, the compound represented by formula (I) (25.0 mg/kg, once a day, 14 days, orally administered), Trametinib (0.3 mg/kg, once a day, 14 days, orally administered), and Adagrasib (10 mg/kg, once a day, 14 days, orally administered) were administered singly or in combination to the mice in a single administration group or a combined administration group. Note that any one of the above agents was not administered to a control group.

Note that, at the time of administration, the compound represented by formula (I) was dissolved in 0.1 M hydrochloric acid at 1.25 mg/mL or 2.50 mg/mL, Trametinib (manufactured by Amadis Chemical) was dissolved in distilled water (pH 8.0) (manufactured by Otsuka Pharmaceutical Co., Ltd.) containing 0.5 w/v% of hydroxypropyl methyl cellulose (manufactured by FUJIFILM Wako Chemicals) and 0.2 w/v% of Tween80 (manufactured by Sigma) at 0.03 mg/mL, and Adagrasib (manufactured by Amadis Chemical) was dissolved in an aqueous solution of 3.5 w/v% of dimethyl sulfoxide (manufactured by Wako Pure Chemical Industries, Ltd.) and 0.5 w/v% of hydroxypropyl methyl cellulose (manufactured by FUJIFILM Wako Chemicals) containing 6.5 w/v% of Tween80 at 1.0 mg/mL.

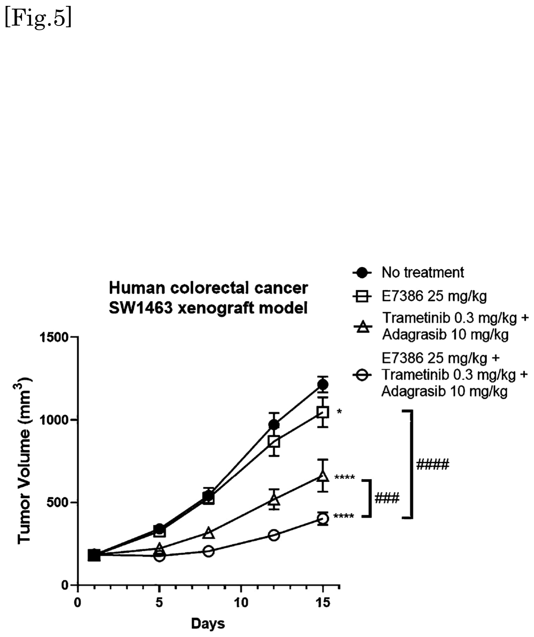

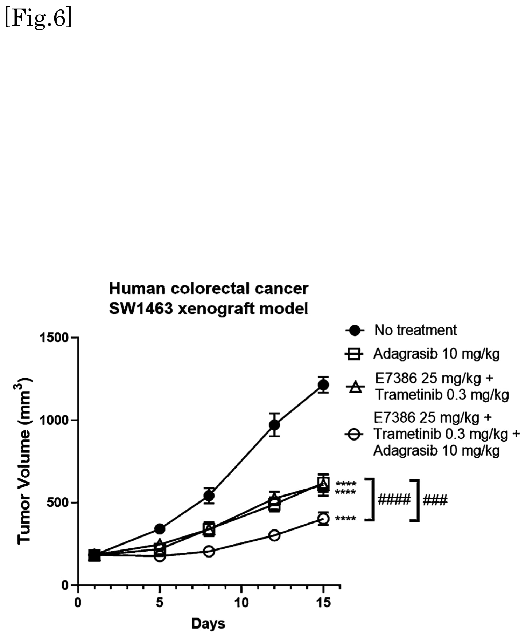

The administration start day was defined as the first day, and a major axis and a minor axis of a tumor generated in each mouse were measured on the fifth day, the eighth day, the twelfth day, and the fifteenth day, and a tumor volume was calculated by a similar method to the above-described method. FIG. 5 illustrates a transition of the tumor volume in a case where 25.0 mg/kg of the compound represented by formula (I) was administered singly, in a case where 0.3 mg/kg of Trametinib and 10 mg/kg of Adagrasib were administered in combination, or in a case where 25.0 mg/kg of the compound represented by formula (I), 0.3 mg/kg of Trametinib, and 10 mg/kg of Adagrasib were administered in three-agent combination. FIG. 6 illustrates a transition of the tumor volume in a case where 10 mg/kg of Adagrasib was administered singly, in a case where 25.0 mg/kg of the compound represented by formula (I) and 0.3 mg/kg of Trametinib were administered in combination, or in a case where 10 mg/kg of Adagrasib, 25.0 mg/kg of the compound represented by formula (I), and 0.3 mg/kg of Trametinib were administered in three-agent combination. Note that, in the drawings, the compound represented by formula (I) is described as E7386. In addition, a measurement result of the tumor volume of each group is indicated as a mean ± standard error (SEM).

As a result, the three-agent combined administration of the compound represented by formula (I), Trametinib, and Adagrasib inhibited tumor proliferation in a statistically significant manner as compared with the case where the compound represented by formula (I) was administered singly or the case where Trametinib and Adagrasib were administered in two-agent combination (FIG. 5). In addition, the three-agent combined administration of the compound represented by formula (I), Trametinib, and Adagrasib inhibited tumor proliferation in a statistically significant manner as compared with the case where Adagrasib was administered singly or the case where the compound represented by formula (I) and Trametinib were administered in two-agent combination (FIG. 6). *, **, ***, and **** in FIGS. 5 and 6 mean p < 0.05, p < 0.01, p < 0.001, and p< 0.0001 versus the control group (Repeated measures ANOVA followed by Dunnett's type multiple comparison), respectively. #, ##, ###, and #### in FIGS. 5 and 6 mean p < 0.05, p < 0.01, p < 0.001, and p< 0.0001 versus the single agent administration group; Repeated measures ANOVA followed by Dunnett's type multiple comparison), respectively.

### [Example 3: Antitumor effect of combined administration of a MEK inhibitor, a BRAF inhibitor, and the compound represented by formula (I) in a transplantation model mouse with human colorectal cancer cell line Colo205 cells]

Human colorectal cancer-derived cell line Colo205 cells (supplied by ATCC) were suspended in Hanks' Balanced Salt Solution (HBSS) (manufactured by FUJIFILM Wako Chemicals) at 5 × 10⁷ cells/mL. 0.1 mL of the obtained cell suspension was transplanted subcutaneously to a right frank of 6-week nude mice (CAnN.Cg-Foxn1 nu/CrlCrlj, manufactured by Charles River Laboratories Japan, Inc.). Six days after the transplantation, a major axis and a minor axis of a tumor generated in each mouse were measured with a digimatic caliper (manufactured by Mitutoyo Corp.), and the mice were divided into groups each including six mice such that an average tumor volume was approximately equal. The tumor volume was calculated according to the following formula.Tumor volume (mm3) = tumor major axis (mm) × tumor minor axis2 (mm2)/2

From six days after the transplantation, the compound represented by formula (I) (25 mg/kg, once a day, 14 days, orally administered), Trametinib (0.3 mg/kg, once a day, 14 days, orally administered), and Dabrafenib (30 mg/kg, once a day, 14 days, orally administered) were administered singly or in combination to the mice in a single administration group or a combined administration group. Note that any one of the above agents was not administered to a control group.

Note that, at the time of administration, the compound represented by formula (I) was dissolved in 0.1 M hydrochloric acid at 2.50 mg/mL, Trametinib (manufactured by Amadis Chemical) and Dabrafenib (manufactured by Amadis Chemical) were dissolved in distilled water (pH 8.0) (manufactured by Otsuka Pharmaceutical Co., Ltd.) containing 0.5 w/v% of hydroxypropyl methyl cellulose (manufactured by FUJIFILM Wako Chemicals) and 0.2 w/v% of Tween80 (manufactured by Sigma) at 0.03 mg/mL and 0.3mg/mL, respectively.

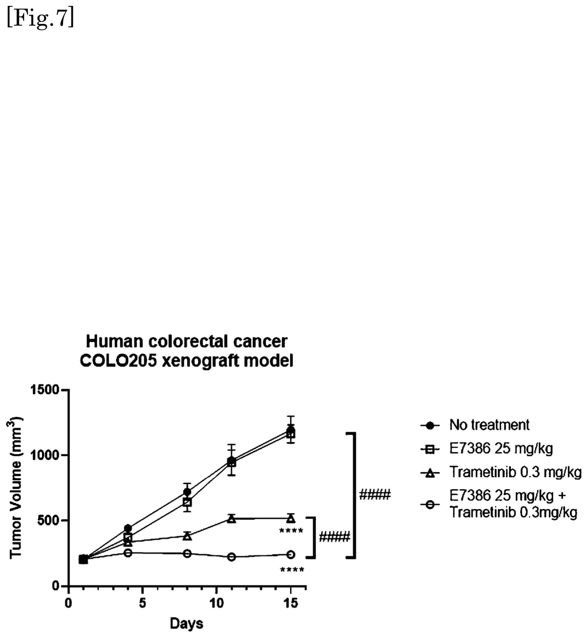

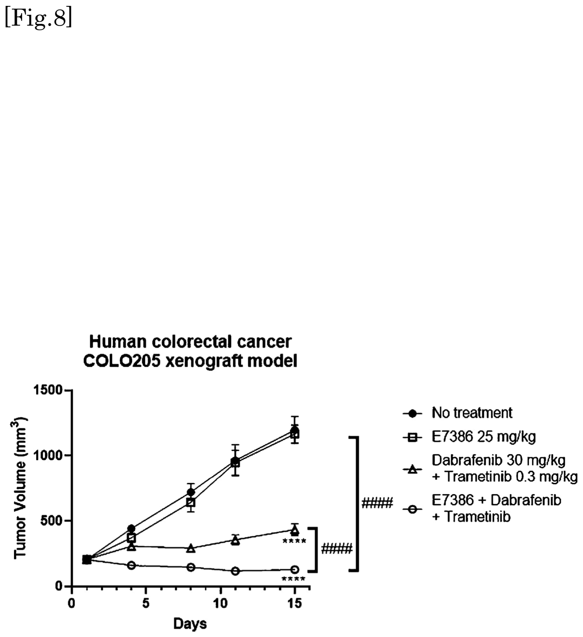

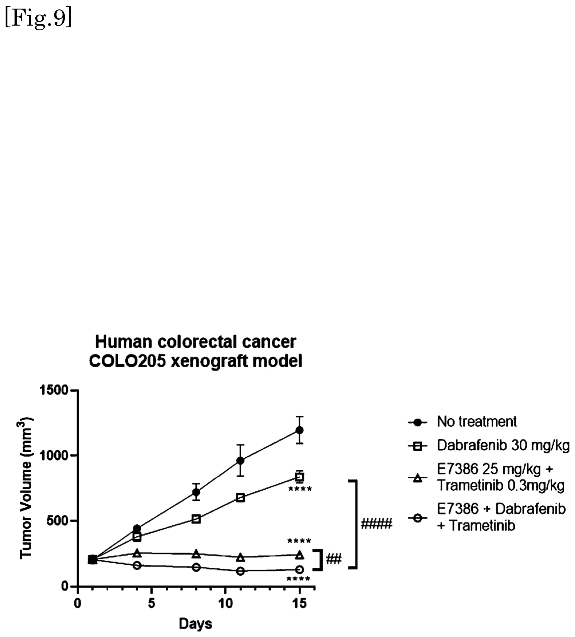

The administration start day was defined as the first day, and a major axis and a minor axis of a tumor generated in each mouse were measured on the fourth day, the eighth day, the eleventh day, and the fifteenth day, and a tumor volume was calculated by a similar method to the above-described method. FIG. 7 illustrates a transition of the tumor volume in a case where 25 mg/kg of the compound represented by formula (I) and 0.3 mg/kg of Trametinib were administered singly or in combination. FIG. 8 illustrates a transition of the tumor volume in a case where 25.0 mg/kg of the compound represented by formula (I) was administered singly, in a case where 0.3 mg/kg of Trametinib and 30 mg/kg of Dabrafenib were administered in two-agent combination, or in a case where 25.0 mg/kg of the compound represented by formula (I), 0.3 mg/kg of Trametinib, and 30 mg/kg of Dabrafenib were administered in three-agent combination. FIG. 9 illustrates a transition of the tumor volume in a case where 30 mg/kg of Dabrafenib was administered singly, in a case where 25.0 mg/kg of the compound represented by the formula (I) and 0.3 mg/kg of Trametinib were administered in two-agent combination, or in a case where 25.0 mg/kg of the compound represented by formula (I), 0.3 mg/kg of Trametinib, and 30 mg/kg of Dabrafenib were administered in three-agent combination. Note that, in the drawings, the compound represented by formula (I) is described as E7386. In addition, a measurement result of the tumor volume of each group is indicated as a mean ± standard error (SEM).

As a result, the compound represented by formula (I) exhibited remarkable enhancement in antitumor effect by the combined use with Trametinib, and inhibited tumor proliferation in a statistically significant manner as compared with the case where the compound represented by formula (I) or Trametinib was administered singly (FIG. 7). In addition, the three-agent combined administration of the compound represented by formula (I), Trametinib, and Dabrafenib inhibited tumor proliferation in a statistically significant manner as compared with the two-agent combined administration of Trametinib and Dabrafenib (FIG. 8). Furthermore, the three-agent combined administration of the compound represented by formula (I), Trametinib, and Dabrafenib inhibited tumor proliferation in a statistically significant manner as compared with the two-agent combined administration of the compound represented by formula (I) and Trametinib (FIG. 9). *, **, ***, and **** in FIGS. 7 to 9 mean p < 0.05, p < 0.01, p < 0.001, and p< 0.0001 versus the control group (Repeated measures ANOVA followed by Dunnett's type multiple comparison), respectively. #, ##, ###, and #### in FIGS. 7 to 9 mean p < 0.05, p < 0.01, p < 0.001, and p< 0.0001 versus the single agent administration group; Repeated measures ANOVA followed by Dunnett's type multiple comparison), respectively.

### [Example 4: Antitumor effect of combined administration of a MEK inhibitor and the compound represented by formula (I), and combined administration of the MEK inhibitor, the compound represented by formula (I), and a KRAS inhibitor in a transplantation model mouse with human non-small cell lung cancer cell line NCI-H358 cells]

Human non-small cell lung cancer cell line NCI-H358 cells (supplied by ATCC) were suspended in Hanks' Balanced Salt Solution (HBSS) (manufactured by FUJIFILM Wako Chemicals) and Matrigel basement membrane matrix (manufactured by Corning International) (1 : 1 mixture) at 8 × 10⁷ cells/mL. 0.1 mL of the obtained cell suspension was transplanted subcutaneously to a right frank of 6-week nude mice (CAnN.Cg-Foxn1 nu/CrlCrlj, manufactured by Charles River Laboratories Japan, Inc.). Seven days after the transplantation, a major axis and a minor axis of a tumor generated in each mouse were measured with a digimatic caliper (manufactured by Mitutoyo Corp.), and the mice were divided into groups each including six mice such that an average tumor volume was approximately equal. The tumor volume was calculated according to the following formula.Tumor volume (mm3) = tumor major axis (mm) × tumor minor axis2 (mm2)/2

From seven days after the transplantation, the compound represented by formula (I) (25 mg/kg, once a day, 14 days, orally administered), Trametinib (0.3 mg/kg, once a day, 14 days, orally administered), and Adagrasib (3 mg/kg, once a day, 14 days, orally administered) were administered singly or in combination to the mice in a single administration group or a combined administration group. Note that any one of the above agents was not administered to a control group.

Note that, at the time of administration, the compound represented by formula (I) was dissolved in 0.1 M hydrochloric acid at 1.25 mg/mL or 2.50 mg/mL, Trametinib (manufactured by Amadis Chemical) was dissolved in distilled water (pH 8.0) (manufactured by Otsuka Pharmaceutical Co., Ltd.) containing 0.5 w/v% of hydroxypropyl methyl cellulose (manufactured by FUJIFILM Wako Chemicals) and 0.2 w/v% of Tween80 (manufactured by Sigma) at 0.03 mg/mL, and Adagrasib (manufactured by Amadis Chemical) was dissolved in an aqueous solution of 3.5 w/v% of dimethyl sulfoxide (manufactured by Wako Pure Chemical Industries, Ltd.) and 0.5 w/v% of hydroxypropyl methyl cellulose (manufactured by FUJIFILM Wako Chemicals) containing 6.5 w/v% of Tween80 at 0.3 mg/mL.

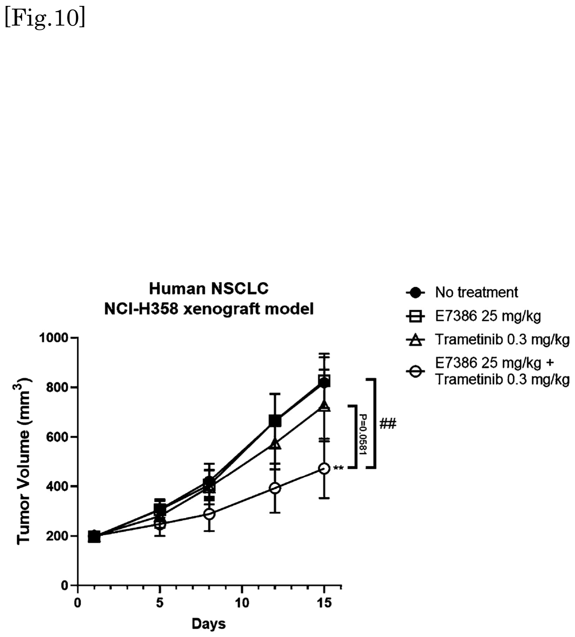

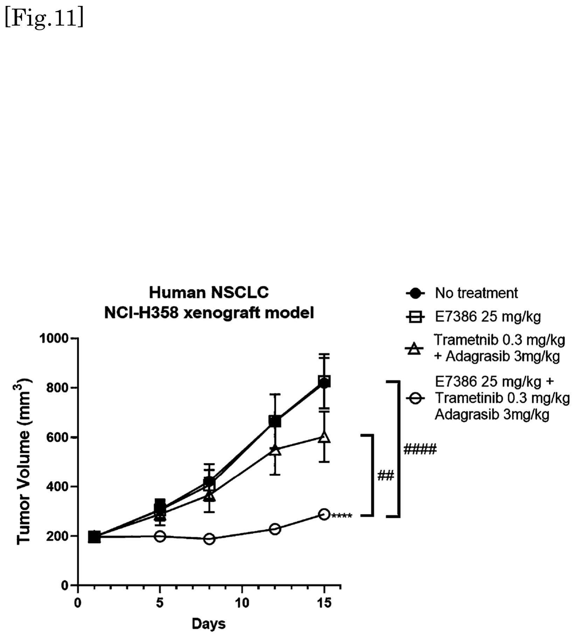

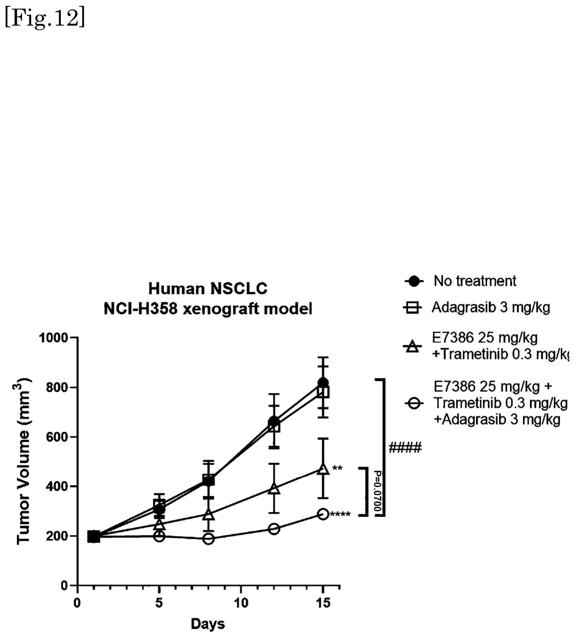

The administration start day was defined as the first day, and a major axis and a minor axis of a tumor generated in each mouse were measured on the fifth day, the eighth day, the twelfth day, and the fifteenth day, and a tumor volume was calculated by a similar method to the above-described method. FIG. 10 illustrates a transition of the tumor volume in a case where 25.0 mg/kg of the compound represented by formula (I) and 0.3 mg/kg of Trametinib were administered singly or in combination. FIG. 11 illustrates a transition of the tumor volume in a case where 25.0 mg/kg of the compound represented by formula (I) was administered singly, in a case where 0.3 mg/kg of Trametinib and 3 mg/kg of Adagrasib were administered in combination, or in a case where 25.0 mg/kg of the compound represented by formula (I), 0.3 mg/kg of Trametinib, and 3 mg/kg of Adagrasib were administered in three-agent combination. FIG. 12 illustrates a transition of the tumor volume in a case where 3 mg/kg of Adagrasib was administered singly, in a case where 25.0 mg/kg of the compound represented by formula (I) and 0.3 mg/kg of Trametinib were administered in combination, or in a case where 3 mg/kg of Adagrasib, 25.0 mg/kg of the compound represented by formula (I), and 0.3 mg/kg of Trametinib were administered in three-agent combination. Note that, in the drawings, the compound represented by formula (I) is described as E7386. In addition, a measurement result of the tumor volume of each group is indicated as a mean ± standard error (SEM).

As a result, the compound represented by formula (I) exhibited remarkable enhancement in antitumor effect by the combined use with Trametinib, and there was a tendency to enhance the antitumor effect as compared with the case where the compound represented by formula (I) or Trametinib was administered singly (FIG. 10). In addition, the three-agent combined administration of the compound represented by formula (I), Trametinib, and Adagrasib inhibited tumor proliferation in a statistically significant manner as compared with the case where the compound represented by formula (I) was administered singly or the case where Trametinib and Adagrasib were administered in two-agent combination (FIG. 11). In addition, the three-agent combined administration of the compound represented by formula (I), Trametinib, and Adagrasib inhibited tumor proliferation in a statistically significant manner as compared with the case where Adagrasib was administered singly, and exhibited an enhancement tendency as compared with the case where the compound represented by formula (I) and Trametinib were administered in two-agent combination (FIG. 12). *, **, ***, and **** in FIGS. 10 to 12 mean p < 0.05, p < 0.01, p < 0.001, and p< 0.0001 versus the control group (Repeated measures ANOVA followed by Dunnett's type multiple comparison), respectively. #, ##, ###, and #### in FIGS. 10 to 12 mean p < 0.05, p < 0.01, p < 0.001, and p< 0.0001 versus the single agent administration group; Repeated measures ANOVA followed by Dunnett's type multiple comparison), respectively.

### [Example 5: Antitumor effect of combined administration of a MEK inhibitor and the compound represented by formula (I), or the combined administration of the MEK inhibitor, the compound represented by formula (I), and a BRAF inhibitor in a transplantation model mouse with human melanoma cell line A375 cells]

Human melanoma cell line A375 cells (supplied by Dainippon Pharma Co., Ltd.) were suspended in Hanks' Balanced Salt Solution (HBSS) (manufactured by FUJIFILM Wako Chemicals) at 5 × 10⁷ cells/mL. 0.1 mL of the obtained cell suspension was transplanted subcutaneously to a right frank of 6-week nude mice (CAnN.Cg-Foxn1 nu/CrlCrlj, manufactured by Charles River Laboratories Japan, Inc.). Thirteen days after the transplantation, a major axis and a minor axis of a tumor generated in each mouse were measured with a digimatic caliper (manufactured by Mitutoyo Corp.), and the mice were divided into groups each including six mice such that an average tumor volume was approximately equal. The tumor volume was calculated according to the following formula.Tumor volume (mm3) = tumor major axis (mm) × tumor minor axis2 (mm2)/2

From thirteen days after the transplantation, the compound represented by formula (I) (25 mg/kg, once a day, 14 days, orally administered), Trametinib (0.3 mg/kg, once a day, 14 days, orally administered), and Dabrafenib (30 mg/kg, once a day, 14 days, orally administered) were administered singly or in combination to the mice in a single administration group or a combined administration group. Note that any one of the above agents was not administered to a control group.

Note that, at the time of administration, the compound represented by formula (I) was dissolved in 0.1 M hydrochloric acid at 1.25 mg/mL or 2.50 mg/mL, Trametinib (manufactured by Amadis Chemical) was dissolved in distilled water (pH 8.0) (manufactured by Otsuka Pharmaceutical Co., Ltd.) containing 0.5 w/v% of hydroxypropyl methyl cellulose (manufactured by FUJIFILM Wako Chemicals) and 0.2 w/v% of Tween80 (manufactured by Sigma) at 0.03 mg/mL, and Dabrafenib (manufactured by Amadis Chemical) was dissolved in distilled water (pH 8.0) (manufactured by Otsuka Pharmaceutical Co., Ltd.) containing 0.5 w/v% of hydroxypropyl methyl cellulose (manufactured by FUJIFILM Wako Chemicals) and 0.2 w/v% of Tween80 (manufactured by Sigma) at 3 mg/mL.

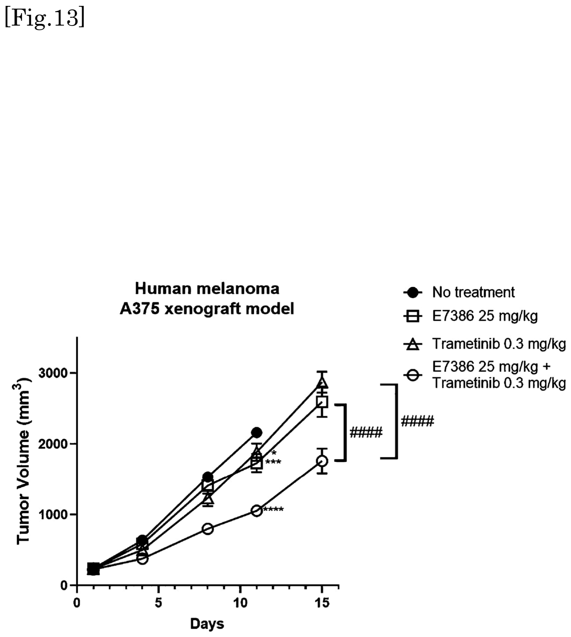

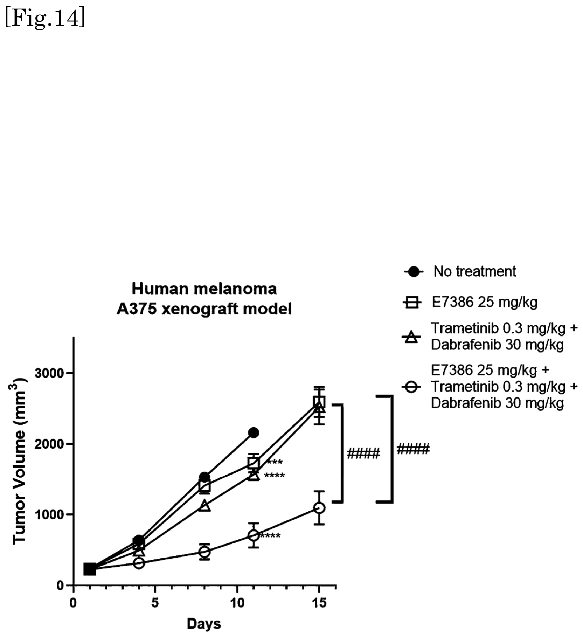

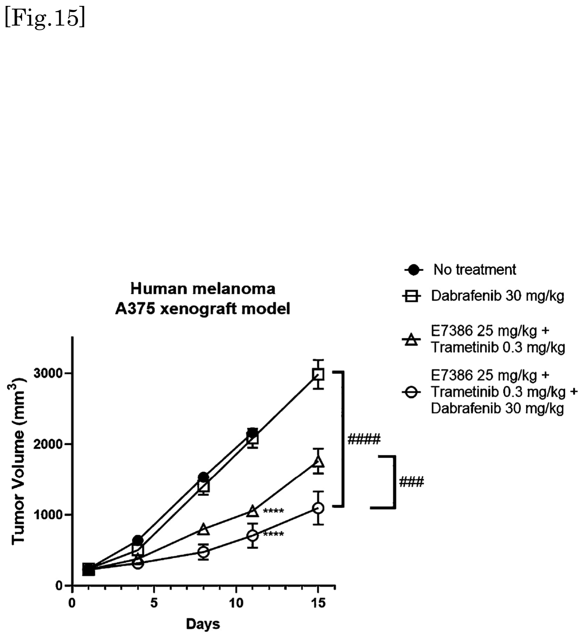

The administration start day was defined as the first day, and a major axis and a minor axis of a tumor generated in each mouse were measured on the fourth day, the eighth day, the eleventh day, and the fifteenth day, and a tumor volume was calculated by a similar method to the above-described method. FIG. 13 illustrates a transition of the tumor volume in a case where 25.0 mg/kg of the compound represented by formula (I) and 0.3 mg/kg of Trametinib were administered singly or in combination. FIG. 14 illustrates a transition of the tumor volume in a case where 25.0 mg/kg of the compound represented by formula (I) was administered singly, in a case where 0.3 mg/kg of Trametinib and 30 mg/kg of Dabrafenib were administered in combination, or in a case where 25.0 mg/kg of the compound represented by formula (I), 0.3 mg/kg of Trametinib, and 30 mg/kg of Dabrafenib were administered in three-agent combination. FIG. 15 illustrates a transition of the tumor volume in a case where 30 mg/kg of Dabrafenib was administered singly, in a case where 25.0 mg/kg of the compound represented by formula (I) and 0.3 mg/kg of Trametinib were administered in combination, or in a case where 30 mg/kg of Dabrafenib, 25.0 mg/kg of the compound represented by formula (I), and 0.3 mg/kg of Trametinib were administered in three-agent combination. Note that, in the figures, the compound represented by formula (I) is described as E7386. In addition, a measurement result of the tumor volume of each group is indicated as a mean ± standard error (SEM).

As a result, the compound represented by formula (I) exhibited remarkable enhancement in antitumor effect by the combined use with Trametinib, and inhibited tumor proliferation in a statistically significant manner as compared with a case where the compound represented by formula (I) or Trametinib was administered singly (FIG. 13). In addition, the three-agent combined administration of the compound represented by formula (I), Trametinib, and Dabrafenib inhibited tumor proliferation in a statistically significant manner as compared with the case where the compound represented by formula (I) was administered singly or the case where Trametinib and Dabrafenib were administered in two-agent combination (FIG. 14). In addition, the three-agent combined administration of the compound represented by formula (I), Trametinib, and Dabrafenib inhibited tumor proliferation in a statistically significant manner as compared with the case where Dabrafenib was administered singly or the case where the compound represented by formula (I) and Trametinib were administered in two-agent combination (FIG. 15). *, **, ***, and **** in FIGS. 13 to 15 mean p < 0.05, p < 0.01, p < 0.001, and p< 0.0001 versus the control group (Repeated measures ANOVA followed by Dunnett's type multiple comparison), respectively. #, ##, ###, and #### in FIGS. 13 to 15 mean p < 0.05, p < 0.01, p < 0.001, and p< 0.0001 versus the single agent administration group; Repeated measures ANOVA followed by Dunnett's type multiple comparison), respectively.

### [Example A-1: Antitumor effect of combined administration of a MEK inhibitor and the compound represented by formula (I) or ICG001 in a transplantation model mouse with human melanoma cell line LOX]

Human melanoma cell line LOX (obtained from ERI) is suspended in Hanks' Balanced Salt Solution (HBSS) (manufactured by FUJIFILM Wako Chemicals) at 5 × 10⁷ cells/mL. 0.1 mL of the obtained cell suspension is transplanted subcutaneously to a right frank of 6-week nude mice (CAnN.Cg-Foxn1 nu/CrlCrlj, manufactured by Charles River Laboratories Japan, Inc.). Seven days after the transplantation, a major axis and a minor axis of a tumor generated in each mouse are measured with a digimatic caliper (manufactured by Mitutoyo Corp.), and the mice are divided into groups each including six mice such that an average tumor volume is approximately equal.

From seven days after the transplantation, the compound represented by formula (I) (12.5 mg/kg or 25 mg/kg, once a day, 14 days, orally administered), Trametinib (0.3 mg/kg, once a day, 14 days, orally administered), and ICG001 (100 mg/kg, once a day, 14 days, intraperitoneally administered) are administered singly or in combination to the mice in a single administration group or a combined administration group. Any one of the above agents is not administered to a control group.

Note that preparation of a drug solution, a method for measuring a tumor volume, and a timing at which the tumor volume is measured are performed in a similar manner to Example 1.

### [Example B-1: Antitumor effect of combined administration of a MEK inhibitor, the compound represented by formula (I), and a RAS inhibitor in a transplantation model mouse with human non-small cell lung cancer cell line NCI-H2122 cells]

Human non-small cell lung cancer cell line NCI-H2122 cells (supplied by ATCC) are suspended in Hanks' Balanced Salt Solution (HBSS) (manufactured by FUJIFILM Wako Chemicals) at 5 × 10⁷ cells/mL. 0.1 mL of the obtained cell suspension is transplanted subcutaneously to a right frank of 6-week nude mice (CAnN.Cg-Foxn1 nu/CrlCrlj, manufactured by Charles River Laboratories Japan, Inc.). Ten days after the transplantation, a major axis and a minor axis of a tumor generated in each mouse are measured with a digimatic caliper (manufactured by Mitutoyo Corp.), and the mice are divided into groups each including six mice such that an average tumor volume is approximately equal.

From ten days after the transplantation, the compound represented by formula (I) (12.5 mg/kg or 25 mg/kg, once a day, 14 days, orally administered), Trametinib (0.3 mg/kg, once a day, 14 days, orally administered), and Sotorasib (manufactured by Angene International, 100 mg/kg, once a day, 14 days, orally administered) as a RAS inhibitor are administered singly or in combination to the mice in a single administration group, a two-agent combined administration group, or a three-agent combined administration group. Any one of the above agents is not administered to a control group.

Note that preparation of the compound represented by formula (I) and the Trametinib drug solution, a method for measuring a tumor volume, and a timing at which the tumor volume is measured are performed in a similar manner to Example 1. Note that Sotorasib is dissolved in 1%Tween80 (manufactured by Sigma)/2%HPMC (manufactured by FUJIFILM Wako Chemicals)/97% distilled water (manufactured by Otsuka Pharmaceutical Co., Ltd.).

A tumor volume proliferation inhibitory effect is confirmed for each single administration group and each combined administration group.

### [Example B-2: Antitumor effect of combined administration of a MEK inhibitor, the compound represented by formula (I), and a RAS inhibitor in a transplantation model mouse with human pancreatic cancer cell line MIAPaCa-2]

An experiment is performed in a similar manner to Example B-1 except that MIAPaCa-2 (supplied by ATCC) is used in place of the human non-small cell lung cancer cell line NCI-H2122 cells as cells to be transplanted into a model mouse.

### [Example C-1: Antitumor effect of combined administration of the compound represented by formula (I), a MEK inhibitor, and a BRAF inhibitor in a transplantation model mouse with human melanoma cell line A375]

Human melanoma cell line A375 (manufactured by ATCC) is suspended in Hanks' Balanced Salt Solution (HBSS) (manufactured by FUJIFILM Wako Chemicals) at 1 × 10⁸ cells/mL. 0.1 mL of the obtained cell suspension is transplanted subcutaneously to a right frank of 6-week nude mice (CAnN.Cg-Foxn1 nu/CrlCrlj, manufactured by Charles River Laboratories Japan, Inc.). Seven days after the transplantation, a major axis and a minor axis of a tumor generated in each mouse are measured with a digimatic caliper (manufactured by Mitutoyo Corp.), and the mice are divided into groups each including six mice such that an average tumor volume is approximately equal.

From twelve days after the transplantation, the compound represented by formula (I) (12.5 mg/kg or 25 mg/kg, once a day, 14 days, orally administered), Trametinib (0.3 mg/kg, once a day, 14 days, orally administered), and Dabrafenib (30 mg/kg, once a day, 14 days, intraperitoneally administered) are administered singly or in combination to the mice in a single administration group or a combined administration group. Any one of the above agents is not administered to a control group.

Note that preparation of a drug solution, a method for measuring a tumor volume, and a timing at which the tumor volume is measured are performed in a similar manner to Example 1.

A tumor volume proliferation inhibitory effect is confirmed for each single administration group and each combined administration group.

## Claims

1. A pharmaceutical composition for treating a tumor, comprising (6S,9aS)-N-benzyl-8-({6-[3-(4-ethylpiperazin-1-yl)azetidin-1-yl]pyridin-2-yl}methyl)-6-(2-fluoro-4-hydroxybenzyl)-4,7-dioxo-2-(prop-2-en-1-yl)hexahydro-2H-pyrazino[2,1-c][1,2,4]triazine-1(6H)-carboxamide represented by the following formula (I) 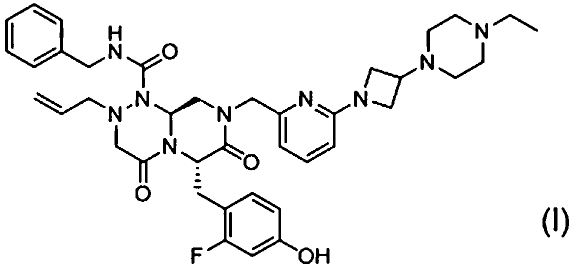 or a pharmaceutically acceptable salt thereof,
wherein the composition is administered in combination with a MEK inhibitor.

2. A pharmaceutical composition for treating a tumor, comprising a MEK inhibitor,
wherein the composition is administered in combination with (6S,9aS)-N-benzyl-8-({6-[3-(4-ethylpiperazin-1-yl)azetidin-1-yl]pyridin-2-yl}methyl)-6-(2-fluoro-4-hydroxybenzyl)-4,7-dioxo-2-(prop-2-en-1-yl)hexahydro-2H-pyrazino[2,1-c][1,2,4]triazine-1(6H)-carboxamide represented by the following formula (I) 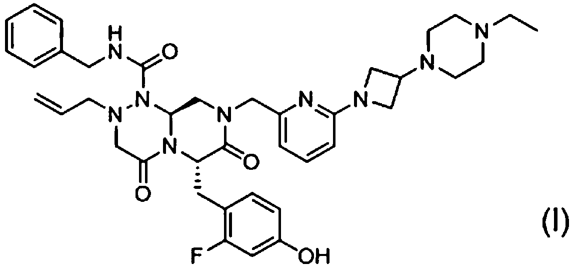 or a pharmaceutically acceptable salt thereof.

3. The pharmaceutical composition according to claim 1 or 2, wherein (6S,9aS)-N-benzyl-8-({6-[3-(4-ethylpiperazin-1-yl)azetidin-1-yl]pyridin-2-yl} methyl)-6-(2-fluoro-4-hydroxybenzyl)-4,7-dioxo-2-(prop-2-en-1-yl)hexahydro-2H-pyrazino[2,1-c][1,2,4]triazine-1(6H)-carboxamide or a pharmaceutically acceptable salt thereof is (6S,9aS)-N-benzyl-8-({6-[3-(4-ethylpiperazin-1-yl)azetidin-1-yl]pyridin-2-yl}methyl)-6-(2-fluoro-4-hydroxybenzyl)-4,7-dioxo-2-(prop-2-en-1-yl)hexahydro-2H-pyrazino[2,1-c][1,2,4]triazine-1(6H)-carboxamide.

4. The pharmaceutical composition according to any one of claims 1 to 3, wherein the MEK inhibitor is selected from Trametinib, Binimetinib, Selumetinib, Cobimetinib, and pharmaceutically acceptable salts thereof; and solvates thereof.

5. The pharmaceutical composition according to any one of claims 1 to 3, wherein the MEK inhibitor is Trametinib or a pharmaceutically acceptable salt thereof, or a solvate thereof.

6. A pharmaceutical composition for treating a tumor, comprising (6S,9aS)-N-benzyl-8-({6-[3-(4-ethylpiperazin-1-yl)azetidin-1-yl]pyridin-2-yl} methyl)-6-(2-fluoro-4-hydroxybenzyl)-4,7-dioxo-2-(prop-2-en-1-yl)hexahydro-2H-pyrazino[2,1-c][1,2,4]triazine-1(6H)-carboxamide represented by the following formula (I) 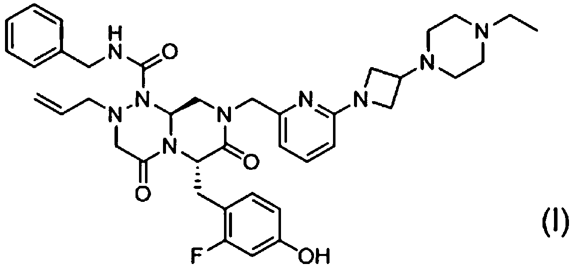 or a pharmaceutically acceptable salt thereof,
wherein the composition is administered in combination with a MEK inhibitor and a RAS inhibitor.

7. A pharmaceutical composition for treating a tumor, comprising a MEK inhibitor,
wherein the composition is administered in combination with (6S,9aS)-N-benzyl-8-({6-[3-(4-ethylpiperazin-1-yl)azetidin-1-yl]pyridin-2-yl}methyl)-6-(2-fluoro-4-hydroxybenzyl)-4,7-dioxo-2-(prop-2-en-1-yl)hexahydro-2H-pyrazino[2,1-c][1,2,4]triazine-1(6H)-carboxamide represented by the following formula (I) 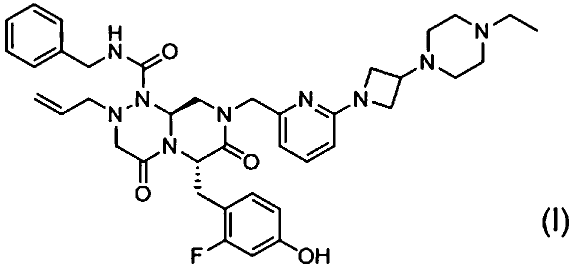 or a pharmaceutically acceptable salt thereof and a RAS inhibitor.

8. The pharmaceutical composition according to claim 6 or 7, wherein (6S,9aS)-N-benzyl-8-({6-[3-(4-ethylpiperazin-1-yl)azetidin-1-yl]pyridin-2-yl}methyl)-6-(2-fluoro-4-hydroxybenzyl)-4,7-dioxo-2-(prop-2-en-1-yl)hexahydro-2H-pyrazino[2,1-c][1,2,4]triazine-1(6H)-carboxamide or a pharmaceutically acceptable salt thereof is (6S,9aS)-N-benzyl-8-({6-[3-(4-ethylpiperazin-1-yl)azetidin-1-yl]pyridin-2-yl} methyl)-6-(2-fluoro-4-hydroxybenzyl)-4,7-dioxo-2-(prop-2-en-1-yl)hexahydro-2H-pyrazino[2,1-c][1,2,4]triazine-1(6H)-carboxamide.

9. The pharmaceutical composition according to any one of claims 6 to 8, wherein the MEK inhibitor is selected from Trametinib, Binimetinib, Selumetinib, Cobimetinib, and pharmaceutically acceptable salts thereof; and solvates thereof.

10. The pharmaceutical composition according to any one of claims 6 to 8, wherein the MEK inhibitor is Trametinib or a pharmaceutically acceptable salt thereof, or a solvate thereof.

11. A pharmaceutical composition for treating a tumor, comprising (6S,9aS)-N-benzyl-8-({6-[3-(4-ethylpiperazin-1-yl)azetidin-1-yl]pyridin-2-yl} methyl)-6-(2-fluoro-4-hydroxybenzyl)-4,7-dioxo-2-(prop-2-en-1-yl)hexahydro-2H-pyrazino[2,1-c][1,2,4]triazine-1(6H)-carboxamide represented by the following formula (I) 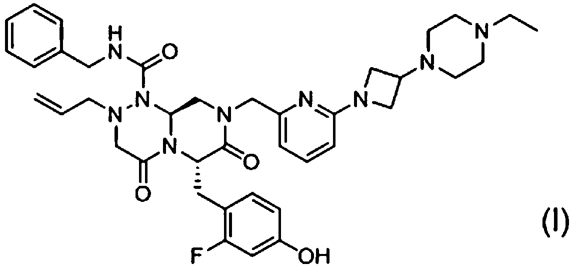 or a pharmaceutically acceptable salt thereof,
wherein the composition is administered in combination with a MEK inhibitor and a BRAF inhibitor.

12. A pharmaceutical composition for treating a tumor, comprising a MEK inhibitor,
wherein the composition is administered in combination with (6S,9aS)-N-benzyl-8-({6-[3-(4-ethylpiperazin-1-yl)azetidin-1-yl]pyridin-2-yl}methyl)-6-(2-fluoro-4-hydroxybenzyl)-4,7-dioxo-2-(prop-2-en-1-yl)hexahydro-2H-pyrazino[2,1-c][1,2,4]triazine-1(6H)-carboxamide represented by the following formula (I) 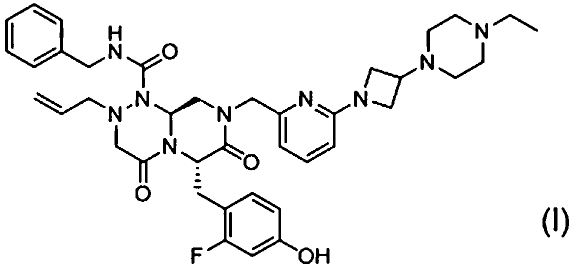 or a pharmaceutically acceptable salt thereof and a BRAF inhibitor.

13. The pharmaceutical composition according to claim 11 or 12, wherein (6S,9aS)-N-benzyl-8-({6-[3-(4-ethylpiperazin-1-yl)azetidin-1-yl]pyridin-2-yl} methyl)-6-(2-fluoro-4-hydroxybenzyl)-4,7-dioxo-2-(prop-2-en-1-yl)hexahydro-2H-pyrazino[2,1-c][1,2,4]triazine-1(6H)-carboxamide or a pharmaceutically acceptable salt thereof is (6S,9aS)-N-benzyl-8-({6-[3-(4-ethylpiperazin-1-yl)azetidin-1-yl]pyridin-2-yl} methyl)-6-(2-fluoro-4-hydroxybenzyl)-4,7-dioxo-2-(prop-2-en-1-yl)hexahydro-2H-pyrazino[2,1-c][1,2,4]triazine-1(6H)-carboxamide.

14. The pharmaceutical composition according to any one of claims 11 to 13, wherein the MEK inhibitor is selected from Trametinib, Binimetinib, Selumetinib, Cobimetinib, and pharmaceutically acceptable salts thereof; and solvates thereof.

15. The pharmaceutical composition according to any one of claims 11 to 13, wherein the MEK inhibitor is Trametinib or a pharmaceutically acceptable salt thereof, or a solvate thereof.

16. The pharmaceutical composition according to any one of claims 6 to 10, wherein the RAS inhibitor is a KRAS inhibitor.

17. The pharmaceutical composition according to claim 16, wherein the KRAS inhibitor is a KRAS G12C inhibitor.

18. The pharmaceutical composition according to claim 17, wherein the KRAS G12C inhibitor is Sotorasib or a pharmaceutically acceptable salt thereof, or a solvate thereof.

19. The pharmaceutical composition according to claim 17, wherein the KRAS G12C inhibitor is Adagrasib or a pharmaceutically acceptable salt thereof, or a solvate thereof.

20. The pharmaceutical composition according to any one of claims 11 to 15, wherein the BRAF inhibitor is selected from Dabrafenib, Encorafenib, Vemurafenib, and pharmaceutically acceptable salts thereof; and solvates thereof.

21. The pharmaceutical composition according to any one of claims 11 to 15, wherein the BRAF inhibitor is Dabrafenib or a pharmaceutically acceptable salt thereof, or a solvate thereof.

22. The pharmaceutical composition according to any one of claims 1 to 21, wherein the tumor is at least one selected from non-small cell lung cancer, colorectal cancer, pancreatic cancer, mesothelioma, uterine cancer, ovarian cancer, bladder cancer, bile duct cancer, stomach cancer, breast cancer, small cell lung cancer, testicular cancer, small intestine cancer, appendix cancer, neuroendocrine tumor, melanoma, and head and neck cancer.

23. The pharmaceutical composition according to any one of claims 1 to 5, wherein the tumor is non-small cell lung cancer.

24. The pharmaceutical composition according to any one of claims 1 to 5, wherein the tumor is colorectal cancer.

25. The pharmaceutical composition according to any one of claims 1 to 5, wherein the tumor is melanoma.

26. The pharmaceutical composition according to any one of claims 6 to 10 and 16 to 19, wherein the tumor is at least one selected from non-small cell lung cancer, colorectal cancer, and pancreatic cancer.

27. The pharmaceutical composition according to any one of claims 6 to 10 and 16 to 19, wherein the tumor is non-small cell lung cancer.

28. The pharmaceutical composition according to any one of claims 6 to 10 and 16 to 19, wherein the tumor is colorectal cancer.

29. The pharmaceutical composition according to any one of claims 11 to 15, 20, and 21, wherein the tumor is colorectal cancer.

30. The pharmaceutical composition according to any one of claims 11 to 15, 20, and 21, wherein the tumor is melanoma.
